# EUROPEAN PATENT SPECIFICATION

(11) **EP 2 617 391 B1**
(45) Date of publication and mention of the grant of the patent: **18.03.2015**
(21) Application number: 13151111.5
(22) Date of filing: 14.01.2013
(51) Int. Cl.: A61F 2/30, A61F 2/08, A61F 2/38

(54) **Anchoring systems for flexible implants for replacing cartilage**
Verankerungssysteme für flexible Implantate zum Knorpelersatz
Systèmes d'ancrage d'implants flexibles pour remplacer un cartilage

(30) Priority: 20.01.2012 US 201213355276
(43) Date of publication of application: 24.07.2013
(73) Proprietor: Formae Inc., Paoli, PA 19301 (US)
(72) Inventor: Dr. Mansmann, Kevin, Paoli, PA Pennsylvania 19301 (US)
(74) Representative: Ettmayr, Andreas

(56) References cited:
- WO-A2-00/74554
- US-A1- 2002 173 855
- US-A1- 2007 224 238
- US-A1- 2009 132 047

## Description

### BACKGROUND

This invention is in the field of surgical implants, and relates to devices for repairing hyaline or meniscal cartilage in joints such as knees, hips, fingers, shoulders, etc.

The background information and prior art can be better understood, if the reader understands the nature and design of the types of implants and anchoring components disclosed herein. Therefore, without digressing too far into a detailed description of the invention in this Background section, a brief overview of several drawings is nevertheless appropriate, at this early stage.

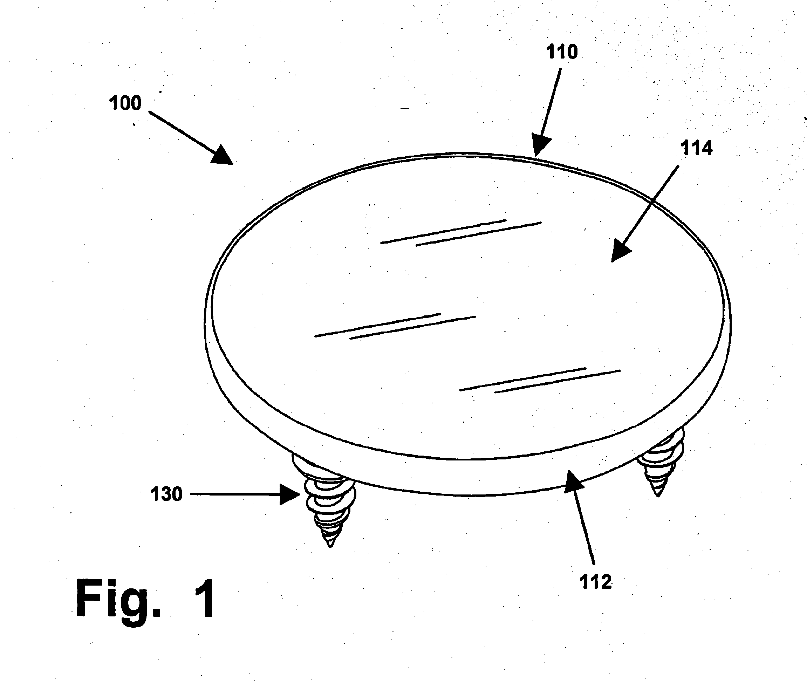

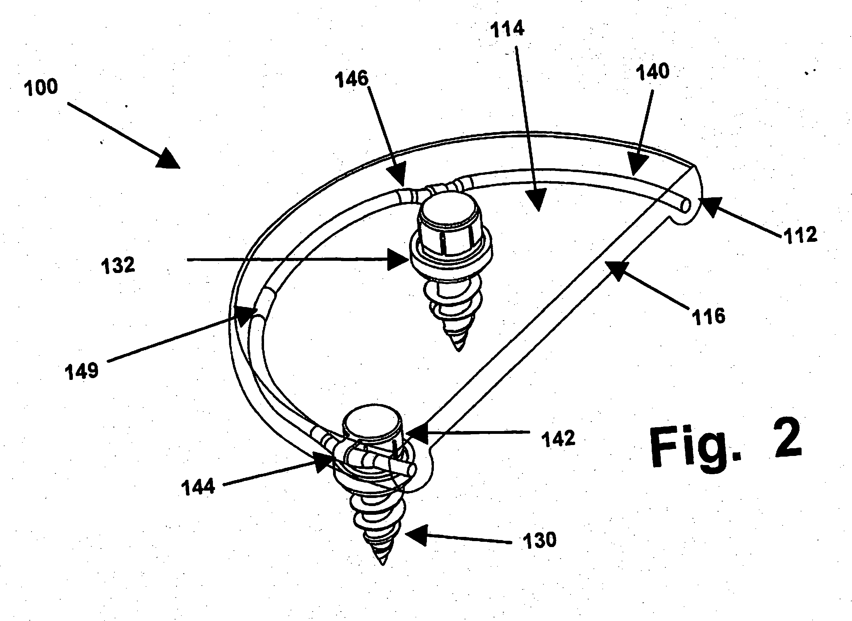

FIG. 1 is a perspective view (and FIG. 2 is a perspective cutaway view) of a cartilage-replacing implant 100. To simplify those two drawings, implant 100 is shown as a simple flat disk, with top side 114 comprising a smooth and wet "articulating surface", which will replace the smooth, wet, slippery surface of a segment of native cartilage, in an articulating joint (which can be a large joint such as a knee, hip, or shoulder, a small joint such as in a finger, or a mid-sized joint such as a wrist or elbow). The term "articulating" indicates that two or more surfaces or components are pressing, rubbing, and sliding against each other.

The "underside" of implant 100, shown as surface 116 in FIG. 2, is called an "anchoring" surface, to reflect the fact that a cartilage-replacing implant must be not just coupled or affixed, but strongly and securely "anchored" to a bone surface (this assumes that the cartilage-replacing implant will be replacing so-called "hyaline" cartilage, which is the type of thin-layer cartilage that covers the articulating surfaces of bones, in a mammalian joint; implants for replacing meniscal or labral cartilage are discussed below). Accordingly, the anchoring surface 116 of implant 100 will be placed in direct contact with a prepared bone surface, from which the native cartilage has been removed (since natural cartilage cannot provide a strong and stable anchoring surface). Initially, implant 100 will be anchored to the bone with the help of 3 anchoring screws 130, which are spaced and distributed around the outer rim (or periphery, or similar terms) 112 of implant 100. Over a span of several months after surgery, bone and/or scar tissue will grow into a layer of porous mesh or similar material that covers anchoring surface 116, to further increase the strength and durability of the anchoring of the implant, to the bone.

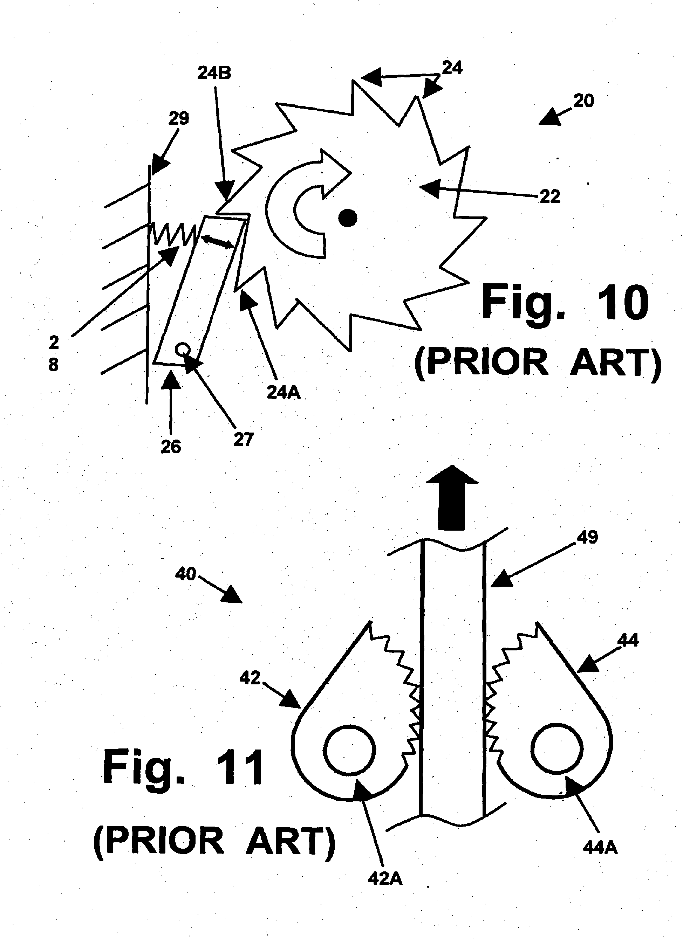

In FIGS. 1 and 2, implant 100 is shown as having a simple round and flat shape, like a pancake or disc. That is solely to simplify the illustrations, so that the various components and structures can be illustrated and understood more simply and clearly. In reality, these types of implants will not be manufactured or used in the form of flat disks, since cartilage segments in human bodies do not have the shapes of flat discs. Instead, these implants will be manufactured in relaxed "unstressed" shapes that emulate and mimic the shapes of the natural cartilage segments they will be used to replace. This is illustrated in FIGS. 10 and 11, in which implant 200 is designed to replace the cartilage on a "femoral runner" in a knee joint. The lower end of a femur bone (i.e., the long bone in the thigh, which extends from the hip to the knee) has two roughly parallel circular segments, called "runners", which press against the top of the tibial plateau and the backside of the kneecap. Each of those two "runners" is covered by a layer of cartilage, and either or both of those two cartilage segments often require surgical replacement.

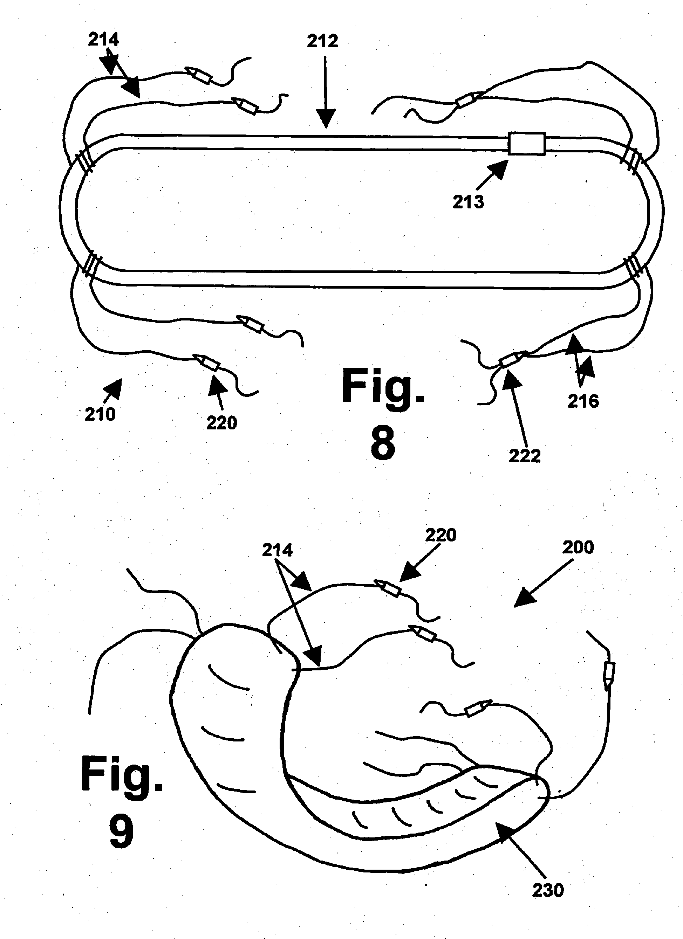

In that type of femoral implant, a flexible anchoring cable 210, which has a generally oval or "racetrack" shape as shown in FIG. 8, will be embedded within the outer rim of a molded polymeric implant 200. The final implant will be molded into a rounded curved shape, as indicated in FIG. 9, which will closely resemble the shape of the natural cartilage segment which covers the surface of a normal femoral runner, in a knee joint.

FIGURES 8 and 9 are also noted, at this early point in the Background section, because they indicate a set of suture strands, which 210, 212, 214, and 216, which will be wrapped around the flexible anchoring cable 210, and which will emerge from the surface of a molded polymer component 230, which will enclose the anchoring cable 210, positioned around the outer rim of the implant device. Those suture strands will pass through a set of "ratcheting knotless suture anchors" 220, which are described in more detail bellow.

Accordingly, when the major anchoring components for these types of cartilage-replacing implants are listed and briefly summarized, they include each and all of the following:
1. a flexible braided or twisted cable, shown as peripheral cable 140 in FIG. 2 and as peripheral cable 212 in FIG. 8, which is embedded within a molded polymeric component around the entire outer (peripheral) rim of the implant devices 100 and 200, shown in FIGS. 1-3 and 9. The outer rim of the molded polymer component will have an enlarged cross-sectional shape 112, as shown in FIG. 2, which is designed and sized to nestle and "seat" in an accommodating manner into a groove or trench that will be machined into a hard surface of the bone that will support the cartilage-replacing implant;
2. a plurality of "snap-cap" devices 142 which are coupled to the flexible peripheral cable 140 at a plurality of spaced locations around the outer rim of implant device 100, as shown in FIG. 2. These "snap-cap" devices are designed to snap securely onto the rounded heads of bone screws that have been driven into a supporting bone. This design will allow the bone screws to be inserted into the joint, and screwed into pilot holes that have been drilled into a bone, before the implant device 100 is inserted into the joint thatg is being repaired, during the surgical procedure. After the bone screws have been installed, the implant device is: (i) inserted into the joint, via an arthroscopic insertion tube; (ii) unrolled or otherwise "opened up" into its final configuration; and, (iii) positioned over the already-installed screw heads. The snap-caps are then pressed firmly against the screw heads, until they snap onto the screw heads, thereby coupling the flexible implant to the bone, via the anchoring screws. This stepwise installation procedure will make it simpler and easier for a surgeon to perform each step in a logical, step-by-step manner, while minimizing the difficulty of each particular step. In particular, a surgeon can remove the damaged or diseased cartilage, prepare the bone surface, and position and emplace the anchoring screws while the operating field remains relatively uncluttered, before the flexible polymeric implant is inserted into the joint.
3. In addition, if desired, a plurality of suture strands can be wrapped around a flexible anchoring cable 140 (shown in FIG. 2) or 212 (shown in FIG. 8), before the polymer component is molded around the anchoring cable 140 or 212. The free ends of the suture strands will emerge, at various spaced locations, from the outer rim of the polymeric implant. This will enable the surgeon to use the suture strands to create additional anchoring points and means for the implant, using either hard bone surfaces, or soft tissues in the vicinity (such as the tendons or ligaments which form the "capsule"-type enclosures which surround articulating joints; these types of joint-surrounding capsules hold in a natural lubricating fluid (i.e., synovial fluid), in a manner which allows the synovial fluid to keep the cartilage surfaces wet and lubricated at all times, in articulating joints.
4. Finally, it should be noted that the free ends of the suture strands 214 and 216, which emerge from the outer rim of polymeric implant 200 as shown in FIG. 9, will pass through specialized types of anchoring devices, referred to herein as "ratcheting knotless suture anchors". Several new types of ratcheting knotless suture anchors have been conceived and developed by the Applicant, as described in more detail below. Those new types of ratcheting knotless suture anchors can enable improved installation and anchoring of the types of implants disclosed herein.

Accordingly, now that the general design and arrangement of these types of implant devices and anchoring components has been introduced, it should become easier for readers to analyze and understand the terminology and prior art, in this specialized field of surgery.

### CARTILAGE AND JOINT TERMINOLOGY

As mentioned above, "articulating" joints, in mammals, are joints in which cartilage-covered surfaces of different bones press and slide against each other. Articulating motion requires the cartilage that is involved to have smooth and slippery surfaces, which are lubricated by synovial fluid. Accordingly, such joints can be called either articulating joints, or synovial joints. This distinguishes articulating joints from spinal joints, and from other cartilage structures in the body (such as in the nose and windpipe) which do not engage in articulating motion (i.e., where different cartilage segments slide across each other).

The types of cartilage that are present in articulating joints are called hyaline cartilage, meniscal cartilage, and labral cartilage. The anchoring systems disclosed herein were developed initially for implants designed to replace hyaline cartilage; however, they can be modified in ways that also will enable their use in meniscal and/or labral implants.

Hyaline cartilage refers to the types of cartilage segments that are affixed directly to bone surfaces, as relatively thin layers. Cartilage-covered bone surfaces are often called condyles; however, that term is used inconsistently. Some people refer to any cartilage-coated bone surface as a condyle, while others limit the term only to cartilage-coated surfaces on elongated bones (which excludes, for example, the socket surfaces in hip and shoulder joints).

Background information on hyaline cartilage, and on surgical implants for replacing injured or diseased hyaline cartilage, is available from various sources, including several published US patent applications by the same Applicant herein, such as serial numbers 11/390539 ("Implants for replacing hyaline cartilage, with hydrogel reinforced by three-dimensional fiber arrays"), 11/105677 ("Hydrogel implants for replacing hyaline cartilage, with charged surfaces and improved anchoring"), and 10/071930 ("Cartilage repair implant with soft bearing surface and flexible anchoring device").

US 2002/0173855 A1 discloses an implant device for repairing damaged cartilage in a mammalian joint comprising a peripheral edge containing an elongated reinforcing member embedded therein and tissue anchoring components coupled to the elongated reinforcing member.

Meniscal cartilage segments (in knee joints) and labral cartilage segments (in hip and shoulder joints) have more complex structures and anchoring systems. Briefly, meniscal and labral cartilage have substantially thicker cross-sections, compared to hyaline cartilage, and they are attached to soft tissues, such as tendons and ligaments, rather than being thin-layer coatings attached directly to underlying hard bone material. As a result, meniscal and labral cartilage segments are made of a specialized type of cartilage called "fibrocartilage", which contains large numbers of exceptionally long and strong bundles of collagen (i.e., the fibrous protein that holds together essentially all connective tissues, in higher animals).

The structures, shapes, and anchoring of meniscal and labral cartilage are similar to each other in numerous respects, and labral cartilage is often referred to as a subtype of meniscal cartilage. That convention is used herein, and any references to meniscal cartilage (or meniscal implants) also apply equally to labral cartilage (or labral implants).

It also must be recognized that the types of cartilage (and types of joints) of interest herein specifically *exclude*:
(1) cartilage in spinal discs. Although made of a specialized type of cartilage, spinal discs do not have any sliding surfaces; instead, they have a very different structures and anchoring means which strongly prevent any sliding motion, since sliding motion between adjacent vertebral bones could severely injure or even sever the spinal cord.
(2) other "non-articulating" cartilage, in various sites such as the nose, ears, windpipe, etc.

The cartilage in spinal discs, and in ears, noses, windpipes, does not need to withstand the types of loads and stresses imposed on articulating joints. Accordingly, implants designed to replace spinal discs, or other non-articulating cartilage segments, do not require the types of anchoring systems disclosed herein. Therefore, prior art implants (and anchoring systems) for replacing non-synovial cartilage segments are not relevant to this current invention, since their functional, structural, and performance requirements are completely different.

All implants of interest herein must be designed to be "substantially flexible", to a point which will enable insertion of any such implant into a targeted joint, via an arthroscopic insertion tube. In most implants, this will involve rolling up or otherwise manipulating an implant into a roughly cylindrical (or arc) shape having a diameter (or width) that is small enough to pass through an insertion tube. The requirement for "substantial flexibility" is described in more detail below, in the Detailed Description section.

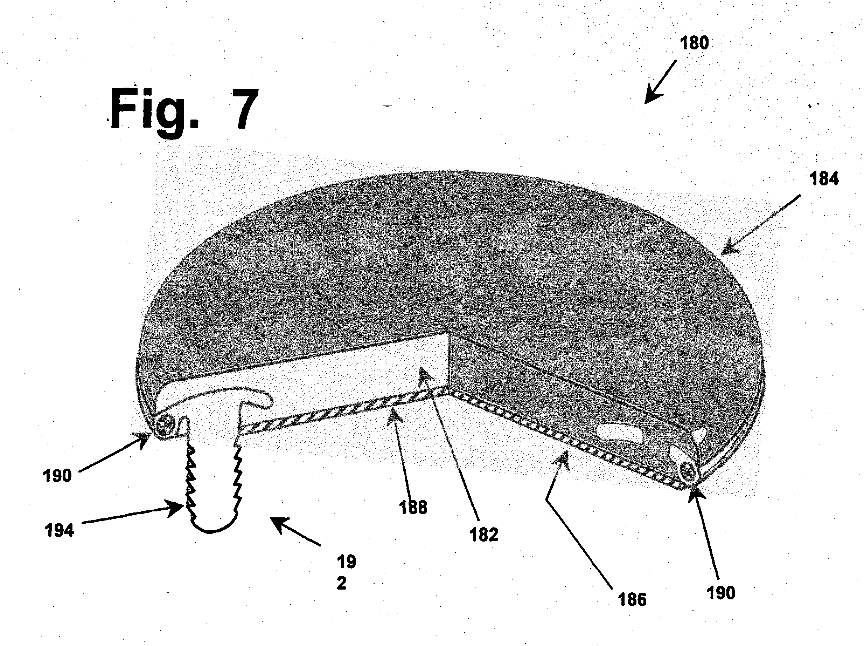

Because the cartilage segments in knee joints are close to the surface and are relatively accessible, initial development and testing of the implants described herein will focus on knee joints, and certain terms that refer to knee structures and surgery merit attention. As illustrated in any textbook on anatomy, a femur (i.e., the long bone in the thigh, between the hip and the knee) has two generally round and parallel runners at its lower end. In each knee, those two runners are designated as the medial (inside) and lateral (outside) runners. In a uni-compartmental repair, only one of those two runners will be replaced or resurfaced; in a bi-compartmental repair (or a "total knee replacement" using classic methods and devices), both of those two runners will be replaced or resurfaced. Accordingly, implant device 200, as illustrated in FIG. 7, is sized and designed to replace a single femoral runner surface. If a surgeon performs "bi-compartmental" resurfacing of both of the femoral runners in a knee, s/he will need to use two such implants.

When a human is standing up, the two femoral runners rest on top of a cartilage surface called the tibial plateau, which generally coats the top surface of the tibia (i.e., the largest bone that extends from the knee to the ankle; a smaller bone that is generally parallel to the tibia is called the fibula, but it does not extend all the way up to the knee joint). The tibial plateau has a small set of raised promontories, collectively called the tibial spine, near its center. Those promontories are not coated with cartilage. Instead, they serve two primary purposes: (1) they occupy the gap between the two round and parallel femoral runners, in a manner which helps stabilize the knee, as the femoral runners travel and slide along the left and right sides of the tibial spine, as the knee is alternatingly bent (flexed) and straightened (extended); and, (2) they provide attachment points for ligaments that extend out from the anterior (front) and posterior (rear) tips of the two meniscal wedges.

The meniscal wedges reinforce the left and right sides of a knee joint, to help prevent sideways (lateral) motion of the femoral runners, on top of the tibial plateau. Accordingly, the meniscal wedges generally encircle the left and right sides of a knee joint, and their tips are coupled, via ligaments, to the bony protrusions that form the tibial spine.

The term "surgery" (and related terms such as "surgical", surgeon, etc.) also requires brief attention. As used herein, the use of needles, injections, or other manipulation of fluids (which can include cell suspensions) does not constitute "surgery", as used herein. As used herein, "surgery" occurs when a skilled practitioner uses a blade, saw, cautery device, or similar instrument to create a cut or incision in one or more types of tissue, or to otherwise manipulate cohesive tissue, as distinct from liquids.

### SHAPE-MEMORY AND SUPER-ELASTIC MATERIALS, AND NITINOL

Since high levels of flexibility will be required for arthroscopic use of the implants disclosed herein, three terms of art in the field of materials science must be addressed. These terms are shape-memory materials, super-elastic materials, and nitinol.

In general, "shape-memory materials" (SMM's, which includes various polymers as well as certain types of alloys) include any materials that fall within either of two somewhat different functional definitions.

Under the first definition, if a material can be deformed (such as by bending, stretching, etc.) in some way that appears to be stable under a first set of conditions, but if the material will return to its manufactured shape, without suffering any permanent damage or alteration, when subjected to a second and different set of conditions, the material can be classified as a "shape-memory material". A common parameter that is used to manipulate shape-memory materials, in ways that make good use of their "shape-memory" trait, is temperature. Temperatures ranges that are of interest for surgical implants must be within a limited and moderate range, and cannot involve temperatures that are so hot or cold that they would injure soft tissues.

For lack of a better descriptive term, the phrase "shape-memory materials" also acquired a second functional definition. If a certain alloy or polymer undergoes some type of "phase transition" which leads to a notably different type of performance or behavior, when subjected to a certain type of operating condition or parameter, and then returns to a "normal" performance or behavior when returned to "normal" conditions, the term "shape-memory material" is often applied, regardless of whether the different performance actually involves shape. This convention apparently arose when it was discovered, during the 1930's, that wires made of certain types of copper-zinc alloys would shrink, in length (which is indeed a change in shape), when heated. Those types of wires came to be used in robotics and toys as "muscle wires", since they will contract, in length, when an electrical current is applied to such wires in a way that causes heating of the wires.

A subsequent development that became medically important arose when it was discovered, in the 1960's, that certain alloys containing nickel and titanium have an unusual behavior. Those alloys were called "nitinol" alloys (pronounced NIGHT-in-all), as a spliced acronym that combines the first letters from nickel, titanium, and "Naval Ordnance Laboratories", the federal research center where nitinol alloys were discovered. Nitinol alloys undergo a temperature-dependent transition that is the opposite of what occurs in most alloys and polymers. Most non-rigid alloys and polymers tend to become softer, and more flexible and pliable, when they are heated to higher temperatures. Nitinol alloys become of medical interest, because they do the exact opposite. At normal body temperatures, nitinol alloys are in an "Austenite" crystalline form, which is relatively stiff. However, if a nitinol device is chilled in cold water (such as "saline slush", a mixture of ice and water containing salt concentrations that make it compatible with cells and tissues), it makes a transition to a "Martensite" crystalline form, which is substantially more flexible and pliable.

As a result of that behavior, various types of medical devices are made of nitinol, such as stents (devices for holding blood vessels open, in people who suffer from partially blocked or occluded arteries such as in the heart or neck). These can be implanted and used as follows. If a stent made of nitinol, in the form of a cylindrical wire mesh, is chilled to a soft and pliable "Martensite" temperature, by immersing it in cold water, the stent can be compressed into a relatively small diameter that will fit inside a catheter tube, which can be "snaked" into a patient's body via a small incision, such as into a femoral artery. The stent can be kept chilled, while it remains in the catheter tube, by using cold water circulating through special channels in the catheter. After the stent reaches a blood vessel that needs to be unclogged, the catheter tube is withdrawn, allowing blood and surrounding tissues to warm the stent back up to its stiffer "Austenite" state. As that warming process occurs, the stent will expand back into its larger, unstressed, manufactured diameter, which will correspond to the inside diameter of the artery segment that needs to be kept open.

These types of nitinol devices, and the transitions they undergo at differing temperatures, are described and shown in more detail in numerous sources, including a website (www.nitinol.info) run by a company called Nitinol Devices and Components (NDC). Several short videos (about 1 minute each), which visually depict how nitinol alloys and devices behave, are available at www.nitinol.info\pages\technology.html. In addition, a review article by D. Stoeckel, "Nitinol Medical Devices and Implants", presented at the SMST 2000 Conference, is available at www.nitinol.info/pdf_files/stoeckel_1.pdf.

Accordingly, nitinol devices will not make self-directed transitions into shorter or longer lengths, or other different shapes, when chilled or heated. However, since they become more pliable and "workable" when chilled, they can be readily manipulated into useful shapes (for an implantation process or other purpose) at cold temperatures, and they will then return to a stiffer and stronger manufactured state and geometry, when allowed to warm up to body temperature. As a result, they are usually included within the class of materials called "shape-memory materials".

The term "super-elastic material" is broader, and it does not have a precise definition. It should be noted that, when used as a scientific term, "elastic" does not simply mean "stretchable". Instead, deriving from their original Greek terms, the words "elastic" and "plastic" form a contrasting pair, comparable to light versus dark, or thin versus thick. The term "elastic" means that if something is deformed, by some external force or condition, it will return to its original shape or status, when the external force or condition is removed. By contrast, the term "plastic" (which can be regarded as a synonym for "moldable") indicates that if something is deformed, it will remain in its newly-created shape or condition.

Clearly, those are functional terms, which depend on conditions. As a simple example, many plastic materials are moldable, and can be given entirely new shapes, by heating them to a temperature which causes the plastic material to soften in ways which allow the molecules to be rearranged; then, when they return to normal temperatures, they become elastic, rather than "plastic".

The term "super-elastic" does not have a precise definition; instead, it includes materials with one or more elastic behaviors that can be especially useful and valuable, in ways that are markedly better than ordinary, when compared to "conventional" elastic materials. In the field of metals, conventional elasticity can be represented by long, thin, flexible pieces of stainless steel, or by the types of steel alloys that are used to make metal springs. In plastics and polymers, conventional elasticity is represented by rubber bands, silicone rubber, etc. Accordingly, "super-elastic materials" include materials that can substantially outperform those conventional materials, in one or more ways that involve elasticity. Since "shape-memory materials" that respond in unusual ways to temperature changes fall within that definition, they are often referred to as super-elastic materials. Other materials with unusual behaviors (such as "muscle wires" that contract, in length, when electric currents are passed through them) fall into a gray zone, where some but not all scientists would refer to them as "super-elastic".

One other point should be noted. In nearly all cases of interest herein, a device made from a shape-memory material usually will seek to return to a certain shape (which will be determined by the manufacturing process), when it returns to a "final" temperature (which will be body temperature, for any surgical implant) or other operating condition. This distinguishes shape-memory devices from items such as rubber bands. A rubber band is elastic, and it will return to a certain length, after any tension that caused it to take an elongated shape has been removed. However, a typical rubber band that has a substantial length will not attempt to return to a certain specific shape. If dropped onto a flat surface, it can come to rest in a relatively straight or oval-like configuration, or it can curve in either a right or left direction, without any substantial stresses arising within the rubber that makes the rubber band.

By contrast, in all cases of interest herein, a shape-memory device will have a predetermined shape, which must be created during a manufacturing operation (which can include various annealing, curing, treating, or other shape-imparting or shape-modifying steps). The device will then seek to return to that predetermined shape. This does not imply that the device must always return to exactly its manufactured shape; nevertheless, it will seek to do so, and any shape alterations that may be imposed on the device, by external mechanisms or forces (such as anchoring pins, an adhesive that is used to bond the material to another surface, etc.), will create some level of internal stresses within the shape-memory or super-elastic device.

Accordingly, proper design of a surgical implant made of a shape-memory or super-elastic material must take into account the final shape that the device will take, after it is implanted in a particular location. Some implants are intended to impose mechanical forces on body parts or mechanical components that contact an implant; this is comparable to installing a spring-loaded device inside a mechanism. However, if creating that type of force is not the intent of a shape-memory or super-elastic implant device, then the implant should be manufactured with an unstressed shape that is as close as possible to the final shape the implant will take, after it has been implanted.

That is a brief introduction to a complex field of materials science. Much more information on these types of materials is available in books (such as Otsuka and Wayman, editors, Shape Memory Materials, Cambridge Univ. Press, 1999), and from an organization called Shape Memory and Superelastic Technologies (SMST), which has a website at www.smst.org. A surgeon does not need to be an expert in this field of materials science, in order to be able to use surgical devices that incorporate and use these types of materials. If a surgeon has a working knowledge of what these materials and devices can accomplish, and how they will perform when used in surgical implants, that is sufficient.

Returning to the subject of nitinol alloys, it was initially believed, by the Applicant herein, that certain types of rims or other anchoring components made of nitinol alloys would be ideal for cartilage-replacing implants, because the use of nitinol alloys would allow them to become more soft and flexible, by using a chilling process during insertion of such an implant into a joint that is being repaired. However, additional research by the Applicant has identified an important obstacle to such use of nitinol alloys, in implants that will remain in a patient's body for an extended period of time. That obstacle involves a risk of corrosion, which is believed to arise primarily in areas where nickel atoms cluster together in "nickel-enriched" clusters or "pockets" that can have molecular structures and/or "lattice ratios" such as Ni₃Ti. The bonds between adjacent nickel atoms are not as strong as the bonds between nickel and titanium atoms. As a result, during the manufacture of a nitinol component, if small pockets of material are formed that have nickel content greater than 50%, the nickel atoms in those pockets can be leached out, over a span of months or years, in ways that can lead to corrosion, cavities, and structural weakness.

It is known that a nitinol manufacturing process known as "Quick Cool with No Reheat" provides more corrosion-resistant nitinol alloys than a different process known as "Cool Down Slowly". Accordingly, nitinol alloys have been approved for use in some medical devices that are left in place for years, such as certain types of stents that help keep arteries open in patients who suffer from clogged arteries.

However, the types of flexible cartilage-replacing implants being developed by the Applicant, for use in load-bearing joints such as hips or knees (where any such implants will need to comply with stricter design requirements and constraints, compared to devices such as stents) already have a number of novel and even pioneering features. Accordingly, this new and innovative "technology platform" is not well-suited for introducing new types of materials that might trigger additional long-term clinical testing requirements. Those types of long-term testing requirements could lead to severe problems and delays, especially if the goal of such long-term trials would be to ensure that a certain type of material will not corrode, over a span of a decade or more, in a mammalian joint.

Therefore, the Applicant began studying alternate types of candidate reinforcing and anchoring devices, using materials with long records of biocompatibility and successful long-term use in implants. The results of those efforts are described below, as part of this invention.

However, it also should be noted that the use of nitinol, in cartilage-replacing implants designed for permanent implantation (in this context, phrases such as "long term" generally refer to time periods greater than 5 years, while "permanent" refers to the remaining life of a patient), might remain as a completely viable approach, if any such nitinol component will be completely embedded within a polymeric material that will effectively "seal in" (or entomb, or similar terms) the nitinol component, in a way that will prevent the nitinol from being contacted, in any significant quantity, by body fluids. That is indeed the design of various types of implants described herein. Accordingly, the use of nitinol anchoring rims, in such devices, remains as a potentially feasible, practical, and approvable design approach, in such implants.

### KNOTLESS SUTURE ANCHORS, AND RatchetING DEVICES

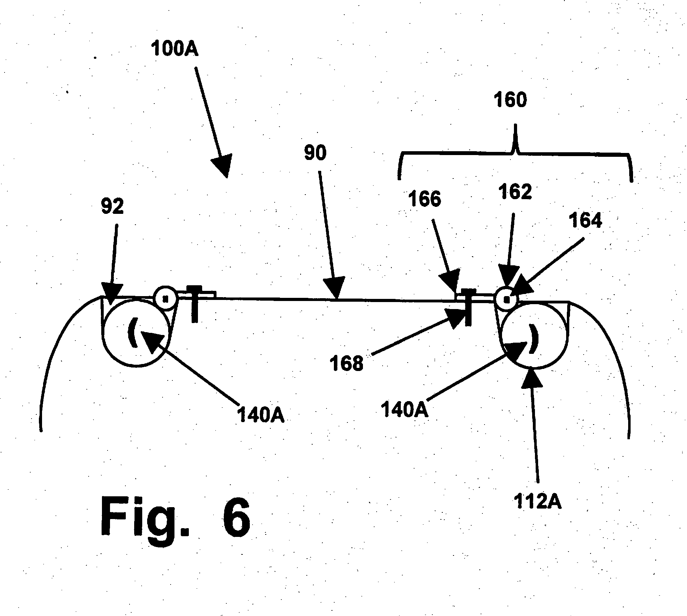

As mentioned above, and as illustrated in FIGS. 6 and 7, one type of anchoring device disclosed herein involves a suture strand which has been wrapped around a flexible anchoring cable that is embedded within a flexible polymeric component of a cartilage-replacing implant. Both ends of the suture strand will emerge from the polymeric component of the implant, in a manner which allows them to be used to help secure the implant, either to a hard bone, or to soft tissue.

Because of the design constraints and installation requirements that will apply to these types of implants, those types of suture strands become especially useful and helpful, if they pass through a type of anchoring device which is referred to herein as a "ratcheting knotless suture anchor". Accordingly, the Applicant herein has developed what are believed to be several new and innovative designs, for new type of miniaturized ratcheting knotless suture anchors which can be used by surgeons to help install and anchor the cartilage-replacing implants disclosed herein.

To help explain why these new devices are believed to be novel, over and above all known items of prior art, the remainder of this Background section focuses on: (1) various types of knotless suture anchors that are disclosed in the prior art; and, (2) various types of ratcheting devices, which also are disclosed in the prior art.

### KNOTLESS SUTURE ANCHORS

Various efforts have been made to design and create surgical devices called "knotless suture anchors", for use in surgery.

A subgroup of such devices, which are of interest herein, are designed to attach soft tissues (such as ligaments, tendons, or muscles) either to hard bones, or to other soft tissues. These types of devices are used mainly by orthopedic surgeons and other specialists, who make every effort to minimize any cutting of (and damage to) any tendons, ligaments, muscles, blood vessels, and other soft tissues that surround injured, diseased, or otherwise damaged or defective tissue, especially in and around articulating joints. Any steps that can be taken to minimize the number and/or lengths of any incisions and cuts that must be made, during arthroscopic or other surgical repair of joints and other structures, are regarded as useful and helpful.

In addition, surgeons are under pressure to work as quickly and efficiently as possible, starting when a patient's skin has been cut or punctured by the first instrument, and lasting until the surgery has been completed, and the incisions have been closed up and covered with one or more bandages. As a general principle, the longer a patient's body or limb stays open, the greater will be the risk, threat, and likelihood of infection.

Accordingly, the requirement of having to tie knots in suture strands, when the only instruments that can be used are long arthroscopic instruments that are narrow enough to pass through arthroscopic incisions that are kept as small as possible, can pose difficult challenges. These challenges become especially complex, when one realizes that typical arthroscopic surgery requires, in addition to the actual surgical instruments, a number of supporting devices, which in most cases will include: (i) a light source; (ii) a camera with a live video feed, which normally will use fiber-optic cables; (iii) a tube which will continuously pump clear saline liquid into the joint or other operating area, to carry blood and debris out of area so that the surgeon can see the structures and tissues that are being manipulated; and, (iv) a drainage catheter or cannula, to suction the saline liquid and its contents out of the joint or body cavity.

Under those conditions, the challenge of tying a knot in a suture strand, especially in a location that may be on the far (distal) side of a bone or other anatomic structure, using only one or in some cases two elongated instruments, can become very difficult, and can be compared to trying to tie a set of shoelaces into a tight and secure knot, using only a single tool, such as needle-nosed pliers.

Surgical staples can be well-suited for securing soft tissues to other soft tissues, but they are not suited for securing soft tissues (or suture strands which have been attached to soft tissues) to hard bone surfaces. When attachments to hard bone are required, more secure devices, usually called anchors (this term includes anchoring screws) are used instead of staples. Some are designed to be screwed or tapped into a "pilot hole" that has been pre-drilled into a bone; others are driven directly into a bone surface, in a manner comparable to driving a nail into a board with no pilot hole.

Accordingly, surgeons and orthopedic supply companies have developed various types of "knotless suture anchors", which enable surgeons to attach suture strands to either hard bones, or soft tissues (different types of knotless anchors are normally used for soft tissues, or hard bones). These knotless suture anchors are described and illustrated in a number of issued patents and published patent applications, which can be divided into several categories, for purposes of analyzing and understanding them.

A first category involves anchors that will undergo some type of shape alteration, after they have been inserted into a drilled hole, in a manner which will cause a set of projections to extend outwardly from the main body of the anchor. For example, the projections will press against or dig into the walls of the pilot hole in a bone, thereby firmly securing the anchor to the bone, and preventing it from being pulled out by any tensile forces which are likely to be imposed on the suture strand. On some of these types of anchors, the projections have spring-type or angled structures that are similar to the "barbs" on a harpoon or fishing hook. On other anchors in this category, the projections are comparable to the types of "expander bolts" that are used to mount large paintings and other heavy wall-hangings to drywall or sheetrock, in homes and other buildings. Issued patents which describe these types of suture anchors include, for example, US 6,328,758 (Tornier et al 2001), US 7,144,415 (Del Rio et al 2006), and US 7,556,640 and 7,695,494 (both by Foerster et al, 2009 and 2010).

A second category of knotless suture anchors includes devices that use two components, which are separate from each other before installation. In nearly all such cases, one component can be regarded as a receptacle, and the other as an insert. In this type of design, the receptacle component is implanted into a bone, normally into a pilot hole. After that component has been inserted, the insert is inserted into the receptacle, typically using tapping, screwing, or similar efforts to drive the insert far enough into the receptacle to lock them together. In some of these anchors, the receptacle component will be fully anchored to the bone, before the insert component is emplaced in the receptacle; in other designs, the act of forcing the insert into the receptacle will cause a shape change which completes the anchoring of the receptacle to the bone.

In these types of knotless suture anchors, a suture strand typically is looped around, passed through, or otherwise coupled or affixed to the insert component, before the insert component is inserted into the receptacle. In some designs, the act of driving the insert into the receptacle will squeeze, crimp, or otherwise secure the suture strand to the anchor, in a manner which cannot be altered later without difficulty; an example of this type of design is provided by US 7,572,283 (Meridew 2009). In other designs, a yielding elastomeric fit between the insert and the receptacle will allow subsequent adjustments to the suture strand, if a tensile force is exerted on the strand which exceeds a "threshold" force; this design is illustrated in several published applications by McDevitt et al, such as US 2003/0130695. Still other designs enable the insert to be manipulated in a way that will allow the receptacle to be removed from the bone, if needed, in case the tension on the suture strand which is held by that anchor needs to be adjusted after an initial fixation; this type of design is illustrated by US 6,540,770 (Tornier et al 2003)

Other designs for knotless suture anchors with various other traits are disclosed in a number of other patents and published applications, which include, for example, US 6,520,980; US 6,585,730; US 7,682,374; and US 7,637,926, all issued to Foerster et al and assigned to either Opus Medical Inc. or ArthroCare Corporation.

A different type of design for ratcheting suture anchors is described and illustrated in two published patent applications, US 2010/0063542 and 2010/0121348, both by Van Der Burg et al. In this design, a suture strand is wrapped around an internal component which can rotate, in a ratcheting manner, within an outer sleeve component. The ratcheting mechanism is provided by a pin, affixed to the top of the rotating internal component, which travels along a sawtooth surface provided by the outer sleeve. The pin can "ride up" each sloped incline on the sawtooth surface. Each time it reaches the top of an incline, it will drop down a steep edge, into a settling location. This effectively prevents the ratchet mechanism from traveling in the non-allowed direction, unless the surgeon takes special steps to disengage the pin from the sawtooth surface so that the tension on the suture strand can be adjusted.

Since this current invention involves different designs for ratcheting suture anchors, the two published Van der Burg applications establish the closest and most relevant prior art known to the Applicant.

The knotless suture anchors described in the prior art are designed (and used, to the extent that any of them are actually used, by surgeons) to reattach tendons, ligaments, membranes, or other soft tissues, to bone surfaces or to other soft tissues. By contrast, the types of suture anchors disclosed herein were conceived and developed as part of an effort to develop a complete system for a very different type of surgical operation, which involves replacing cartilage segments, in joints such as knees, rather than reattaching tendons to bones for purposes such as rotator cuff repair.

Because of certain operating requirements and constraints that arise in the types of cartilage repair operations being developed by the Applicant herein, he began with a completely different design, compared to the approach disclosed by Van der Burg et al. Subsequently, after locating and reviewing the Van der Burg applications, the Applicant herein realized that there are major differences in the two approaches to creating ratcheting suture anchors, and that the designs disclosed herein can offer a number of advantages, compared to Van der Burg's approach, when used to anchor cartilage-replacing implants.

When the Applicant herein began to focus on the details of how a set of suture strands can be affixed to bone surfaces, to help anchor a flexible implant that can replace damaged cartilage, his attention turned to knotless suture anchors, and when he realized that none of the knotless anchors that are currently known would be optimal for the particular use he intended, he began to focus on how new and different types of knotless suture anchors could be designed, which would be optimized for that particular type of intended use.

Those analyses led him to conclude that a new design for "ratcheting" suture anchors can provide substantial improvements over all other currently known types of "ratcheting" or other suture anchors.

A full understanding of the preceding sentence will require a working knowledge of ratchet mechanisms in general.

### RATCHET MECHANISMS

Some sources assert that "rachet" is a proper spelling for the types of devices discussed herein; however, "ratchet" apparently has become preferred, and is used herein.

In addition to having two different spellings, the term "ratchet" has acquired two different meanings. Those different meanings can lead to confusion, if not adequately understood.

A relatively narrow "classic" definition of "ratchet", used by specialists such as mechanical engineers, requires the presence of both a gear, and a "pawl". This type of ratchet mechanism 20, which has been known for centuries in the prior art, is illustrated in a simplified form in FIG. 10, which is prior art, and which shows a rotating gear 22 having surface protrusions 24 (often called teeth, cogs, or similar terms). Under the narrow and classic definition of ratchet, a ratcheting mechanism must also contain a "pawl" 26, which refers to any type of mechanism that will engage the teeth of the rotating gear in a manner that allows rotation in one direction, but not the other direction.

The designs of various types of interactive gears and pawls can become complex and sophisticated, and FIG. 10 is a simplified depiction of a basic mechanism. The pawl 26 is mounted on its own axle 27, and the operating end of pawl 26 is pressed against the teeth of gear 22 by the action of spring 28, which is mounted against a relatively stationary surface 29. The external spring is shown, solely for purposes of illustrating the basic arrangement; in nearly all types of pawl systems in use today, an internal (and therefore protected and unintrusive) coil spring is coupled to the axle of the pawl, to provide the same effect.

In a "classic" ratchet mechanism, the positioning and movement of the pawl constrains the travel of the gear, in a manner which allows the gear to rotate in only one direction. If a rotational force drives the gear to rotate in the direction shown by the block arrow in FIG. 10, the surface of gear tooth 24a will press against the side of pawl 26, in a way which will deflect pawl 26, causing it to rotate slightly about its axle 27 while spring 28 is compressed slightly. This allows gear tooth 24a to move "forward" and occupy the position currently occupied by tooth 24b in FIG. 10, which presses directly against the end of pawl 26.

A properly-designed pawl will not deflect and temporarily move out of the way, if the lower surface of gear tooth 24b presses against the end surface of pawl 26. In the depiction in FIG. 10, the axle-mounted placement of the pawl will allow the pawl to be deflected in a "sideways" (i.e., left-and-right) manner, but it will not allow the upper end of pawl to move in a "downward" direction. This is comparable to saying that if a conventional wagon is sitting on a sidewalk, it can be pulled horizontally, with relatively little effort, and it will simply roll, because of how its wheels and axles function. However, that same wagon cannot be pressed downward, into the sidewalk, without damaging and effectively breaking the wagon.

Ratchet mechanisms of this type are common and well-known. If desired, they can be modified in various ways, to adapt them for additional purposes. For example, in a so-called "ratchet wrench" (or ratcheting screwdriver), a V-shaped pawl with two arms can be mounted next to a gear, using an axle component that will allow either one arm of the V-shaped pawl, or the other arm of the pawl, to engage the toothed gear at any particular time. In this way, operation of an external lever or other control device will allow the user of a ratchet wrench (or screwdriver) to set the tool in a first configuration that will tighten a bolt, nut, or screw when desired, and to subsequently change the setting of the wrench or screwdriver, so that it can loosen a bolt, nut, or screw.

Alternately, a ratchet wrench or screwdriver can have two separate and independent pawl components, and an external control lever will rotate an internal component which can push either pawl out of engagement with the gear, while allowing the other pawl to move into contact with the gear and engage it.

Accordingly, in the relatively narrow "classic" definition, a true "ratchet" system requires a gear, and at least one pawl component which can engage and constrain the gear in a manner that allows the gear to rotate in only one direction for as long as that pawl engages the gear.

However, a broader definition has emerged, which is widely and commonly used, and which is preferred and used herein. Since most users do not know or care what type of mechanism is being used to create a ratcheting effect, the term "ratchet" has come to include any mechanical linkage which allows motion in one direction (which can be linear, rotational, or any combination), while preventing motion in the "other" direction (which can be called the opposite, prohibited, blocked, or non-allowed direction, or similar terms).

Yet another uncertainty can arise, in determining whether the term "ratchet" should:
(1) be strictly and narrowly limited, so that it applies only to devices and systems having mechanisms which completely block and prohibit motion in a "non-allowed" direction; or,
(2) be used in a more expansive and tolerant manner, to also include devices which can impede (or "strongly impede") motion in a non-allowed direction, at a level which is sufficient to generally prevent such motion, but which can yield, in a manner that will prevent damage and destruction, if the force that attempts to drive a ratchet in a non-normal direction exceeds some type of threshold.

The types and classes of mechanisms which dwell in that zone of uncertainty, where it is not clear whether they do or do not properly and accurately qualify as "ratchet" devices or system, is illustrated and exemplified by the type of belt buckle that is often called a "cinch buckle". This type of buckle, which is often found on woven or braided belts that are used to hold up trousers (cinch buckles normally are not used with leather belts or straps, since they would damage the leather), involves two metallic rings which are adjacent or close to each other, where they effectively become "parallel" circles or arcs. Each metal ring will have a portion (which can be a straight segment, within an otherwise circular ring) that is constrained within the webbing or fabric of the belt. When the free end of a belt is looped through a "cinch buckle", the act of looping the belt over and around the "top" ring, before lacing it back through the lower ring and then pulling it tight (so that the rough or textured surface of a woven or braided belt will be pressed against itself) creates a squeezing and crimping force which pulls and presses the upper ring (and its loop of belt material) downward against the lower ring. In this manner, the two adjacent metal rings can squeeze and effectively grab a woven or braided belt, with sufficient strength to allow the belt to function adequately, in holding up trousers.

Accordingly, a cinch buckle can qualify as a ratcheting device, under a broad definition of "ratchet", since it allows one end of a belt or strap to be pulled in one direction (i.e., in a tightening direction), and it then generally prevents that end of the belt or strap from traveling in the opposite direction (which would quickly loosen the belt or strap).

However, the fact that a cinch buckle can only *generally* prevent travel of a belt or strap in a non-allowed direction requires attention, because a cinch buckle does not have any mechanism which truly *prohibits* the type of non-desired travel that is referred to by terms such as slippage, creep, etc. In general, a belt with a cinch buckle is adequate for holding up trousers, only if the person wearing the belt is able to conveniently and discretely reach down and tighten the belt when necessary to do so, during the course of a day or evening, each time the belt becomes too loose to function effectively. If desired, the surfaces of the rings can be have knurled or other rough or textured surfaces, which can help reduce slippage, but those types of steps do not change the nature of a cinch buckle.

To a large extent, the proper use of terms such as "ratchet" will depend on the setting, functional requirements, and context of the usage. For example, a cinch buckle might properly and reasonably be referred to as a ratchet mechanism, if used to secure a belt around a duffel bag or comparable item that is being used to store or transport clothes or other lightweight items.

However, a cinch buckle cannot be used to safely secure heavy cargo to a flatbed trailer, in the types of 18-wheeler trucks that haul cargo across highways. Since the risk of a cinch buckle gradually losing its "grip" on a strap or belt is so high, in an environment where vibration, jostling, or other repetitive motion occurs (and where unintended release of the cargo, from a truck driving at high speed down a highway) might kill or maim innocent people, it would constitute reckless disregard (or even criminal neglect) if a trucking company used "cinch buckles" on nylon straps to secure heavy cargo to truck trailers. Accordingly, in that type of setting, a cinch buckle would not qualify as a ratchet mechanism.

Before moving on to a class of ratchet devices called "cam cleats", it also should be noted that various types of ratcheting systems, devices, and designs are known, where it is not clear whether some particular mechanism does, or does not, comprise a gear-and-pawl system. As one example, in various types of devices (such as child-proof caps on pill bottles, in the lids of plastic pails that hold chemicals for swimming pools, etc.), a cylinder, disc, cap, or other rotatable component can be provided with a protruding "flap" or ramp-like structure on its periphery. When provided on the cap or lid of a container, that ramp-like structure usually is designed to rub against (and move across) a series of accommodating slots or ridges, which have been molded into the neck of the bottle, jar, pail, or other portion of the container, when the cap or lid is being tightened. Subsequently, if someone tries to remove the cap or lid, by rotating it in the opposite direction, the ramped structure on the cap or lid will "catch" on the slots or ridges of the bottle or pail, in a manner which will prevent rotation, unless certain additional steps are taken. Accordingly, this type of "safety" cap or lid can prevent a toddler from opening a bottle of pills, and it can prevent a pail of chemicals from coming open accidentally.

The point that should be recognized, in analyzing what might or might not qualify as a "true" or "classic" ratchet, is that some mechanical engineers would label the protruding component on such a cap or lid as a "pawl", and would label the ridged or slotted components on the container as a "gear" (or gears), but other mechanical engineers likely would not agree that those "classic" terms should be stretched far enough to cover those types of devices.

Similarly, in the system illustrated in US application 2010/0063542 (Van Der Burg et al), a pin, which projects outwardly from a rotating internal component, interacts with a sawtooth surface on top of a cylindrical sleeve which surrounds the internal member. Similar systems are widely used in retractable ballpoint pens, to allow an endless number of extensions and retractions of the ink point, by repeatedly pressing a button-type device mounted on top of the barrel of the pen. Some mechanical engineers might regard Van Der Burg's pin mechanism (and/or the mechanism in a conventional retractable ballpoint pen) as a "classic" gear-and-pawl system, while others probably would not.

As shown by the various examples above, the narrow definition of "ratchet" systems (i.e., as being limited to "gear and pawl" systems) is not merely limiting, it is uncertain, potentially confusing, and difficult to apply and use consistently, when one realizes how many borderline cases might or might not be covered by the narrowly-defined "classic" definition. Therefore, the broader definition (i.e., which includes any mechanism designed to allow travel of some component in one direction, while generally prohibiting and preventing travel of that component in the opposite direction) is clearer, and makes better logical and practical sense, and is preferred and used herein.

One example of ratchet linkages other than the classic "gear and pawl" linkage is provided by devices called "cam cleats", which are commonly used on sailboats to temporarily secure ropes in certain positions. A cam cleat is generally depicted in FIG. 11. Better illustrations, such as photographs of actual devices, are readily available in the online catalogs of companies that sell sailboat equipment.

The term "cleat" has been used for centuries, to refer to certain types of devices which are mounted on sailboat rails, and on docks, piers, and similar locations. Cleats are designed to enable ropes to be secured to them, without requiring a rope to be tied into a knot; alternately, if a knot is used to create a loop at the end of a rope, then that loop will effectively become a permanent part of that rope, and the knot will not need to be tied, and then untied, for each "cycle" of use.

There are powerful reasons, in sailing, for not wanting to have to repeatedly tie and untie knots in ropes. When large pulling forces are exerted on any knot (as often occurs whenever boats are involved, due to waves, tides, wakes from other boats, etc.), a knot that has been subjected to even a single moment of a large tensile force can be compacted into a very tight and hard configuration. It can be very difficult (or effectively impossible) to untie a knot which has been tightened to an extreme level of tightness and hardness, without tedious and extensive effort. Therefore, "cleats" were developed and designed as mechanisms that allow ropes to be secured to them, without requiring those ropes to be tied into knots.

In mechanical terms, "cam" refers to devices which generate some type of linear motion or travel when they rotate. This is usually accomplished by either of two types of designs. In one design, a gear or similar rotatable component (which might have either a smooth surface, or a toothed, textured, or other non-smooth surface), which has a genuinely circular shape, is affixed to a rotating axle, in some location other than the center of the gear,. This creates an "eccentric" mounting of the gear, on the axle. As a result, each time the gear rotates through a complete revolution while the axle is constrained, the "apparent" surface (or thickness) of the gear, when viewed from some particular angle, will generate a reciprocating (i.e., back-and-forth) linear motion, which can be imparted to a device such as a spring-mounted linear component.

The other main type of design for cam devices uses a rotating shape which is not truly circular. An example is provided by the "camshaft" devices used by automobile engines. A typical "cam gear" of this type has roughly the same elongated shape as a chicken egg, so that each time the camshaft rotates through a cycle, the "point" of each cam gear mounted on the camshaft will cause an engine valve to be displaced slightly, in a manner that will briefly open that particular engine valve. The inlet valves allow fuel or oxygen to enter a cylinder, in a manner that is precisely timed and controlled by rotation of the numerous non-circular gears on the camshaft, while the outlet valves allow the hot exhaust gases to exit the cylinders, at carefully synchronized moments in time.

Regardless of which type of design is used, cam devices are designed to cause "translational" (linear) motion of a surface which can rotate about an axle. Some cam devices make complete and multiple rotations (such as automobile camshafts), while other types of cam devices never complete a full rotational cycle.

A typical cam cleat, on a sailboat, has two gears, and neither gear is able to rotate through an entire circle. As indicated by the cam cleat mechanism 40, as shown in FIG. 11 (which is prior art), the two gears 42 and 44 are mounted on axle components 42a and 44a. Each axle incorporates a spring-loaded mechanism, to constantly exert a low-level force on each of the gears 42 and 44, which will constantly try to close the two gears together. The spring-generated force which attempts to close the two gears against each other will ensure that the ridges or "teeth" 42b and 44b of the two gears 42 and 44 will continually be pressed against the surface of rope 49, which passes between the two gears.

For simplicity of illustration, the surfaces of rope 49 are shown as being smooth. In practice, any such rope (usually braided from multiple strands of nylon or polypropylene) will have a rough or textured surface, which will enable a better "grip" by a cam cleat. A "monofilament" rope (as used in fishing lines, to make it harder for fish to see a line attached to a lure or bait) would not be used in this type of setting.

Because of the design and arrangement of cam cleat 40, as illustrated in FIG. 11, rope 49 can be pulled through cam cleat 40 in one direction, shown by the block arrow, with little or no resistance. However, if the rope tries to travel in the opposite direction, through the cam cleat, the teeth 42b and 44b on the non-circular cammed surfaces of the two gears 42 and 44 will "bite into" the rope, in a manner which prevents travel of the rope in the "blocked" or prohibited direction. As the teeth on the two gears 42 and 44 rotate slightly in the "not allowed" direction, due to a pulling action exerted by the surface of the rope, the ridges of those surfaces will be pulled closer together, because of the non-circular cammed shape of gears 42 and 44. This will cause the gear teeth to "bite" even harder into the rope. This generates a powerful squeezing and gripping force, and if the rope is pulled even harder, the gears of the cam cleat will be pulled even closer together, causing the cleat to grip the rope even more tightly than before.

In a typical cam cleat on a sailboat, the cam cleat will have either: (1) an open top surface, to allow someone to quickly release the rope from the cleat, by jerking the rope in an upward direction, at a location near the cleat; or, (2) a specialized constraining bracket, which will require the rope to be pulled upward in a specific manner, before the rope will be released by the two cam gears. That type of constraining bracket can reduce the risk of accidental release of a rope at an unwanted and possibly dangerous time.

The risk of accidental release of a rope, by a cam cleat, merits attention. In general, on sailboats, cam cleats without adjacent fixed cleats are used only for temporarily securing ropes that are called "sheets". This set of ropes is used to trim the sails (i.e. they are used to pull sails and booms in horizontal directions). By contrast, any ropes that are used to raise or lower sails or booms (or other devices), in a vertical direction, are referred to as "halyards". The distinction between "sheets" and "halyards" is crucially important, and is taught in any beginning class on sailing.

Halyards are not used nearly as frequently as sheets, and a sudden failure of a halyard would be more likely to cause a serious and perhaps catastrophic problem or failure, up to and including sinking of a boat, and loss of life. Therefore, if a cam cleat is included in the mechanism that is used to raise a halyard on a small sailboat, a fixed cleat can be positioned next to the cam cleat. This arrangement will allow a sailor to get a secure grip on a halyard, pull hard on it to raise a sail a limited distance, and then let go of the halyard for a moment, in order to grab the halyard at a spot closer to the mast, to provide a better grip and better leverage for the next tug on the rope. Accordingly, the type of ratcheting control that is provided by a cam cleat allows someone to raise a sail all the way up a mast, by means of a series of short pulls on a halyard rope. Once the sail has been raised, the halyard is secured to a fixed cleat mounted next to the cam cleat, to ensure that the rope cannot be released accidentally.

Alternately, a sailor on a small sailboat can simply wrap the free end of a halyard rope around the mast, and lightly tie the rope to the mast, using a simple knot. The act of securing the rope close to the mast will effectively cause the rope to remain near the bottom of the cam cleat gears, and will help ensure that the rope will not be lifted and raised, somehow, out of the grip of the gears in the cam cleat.

In contrast to halyards, which raise and lower things vertically on a boat, cam cleats are frequently used to pull and secure "sheet" ropes on a sailboat, despite the well-known and well-recognized risks that cam cleats (especially "open top" cam cleats) sometimes fail. Skilled sailors must learn to accept and respect those risks; for example, if they hear a suspicious sound which indicates that something might be going wrong, they are taught to duck, immediately, rather than stand up and look around, in case a cam-cleat has failed and has allowed a fast-moving boom to swing around unexpectedly. There are plenty of references to sailors "taking swimming lessons" if they fail to recognize and respect the risk that a cam cleat might fail and release a rope it was holding.

Other types of mechanical ratcheting systems are also known. For example, some types of cam cleats have a single non-circular gear which can rotate; when the rope attempts to pull that gear in the non-allowed direction, the teeth on the non-circular gear will press the rope harder and harder into a constrained channel which has non-moving but ridged gripping surfaces. These types of single-gear cam cleats can be found on adjustable bungee cords and various other devices.

### ADVANTAGES OF RATCHETING ANCHORS FOR SECURING CARTILAGE IMPLANTS; START-SNUG-TIGHTEN PROCEDURES

When used to help anchor and reinforce surgical implants that are designed to replace damaged cartilage, one of the advantages that could be provided by ratcheting suture anchors - if such devices are developed and manufactured with sufficiently high levels of reliability, and sufficiently low risks and rates of failure -- is that they would enable a surgeon to perform a type of installation procedure that would be very useful.

Those three steps can be summarized by the phrase, "start them all, then snug them all, then tighten them all".

If desired, that phrase can be shortened to "start, snug, tighten", so long as the reader understands that the entire "start" procedure must be finished for all of the sutures, before the second procedure should be started for any of the sutures.

An example of how this approach functions, in a different field, involves replacing a flat tire on an automobile. After the car has been jacked up, to remove the weight from the flat tire, the wheel (i.e., the metal or graphite "hub", with the flat tire still affixed to the hub) is pulled off of an assembly called a "wheel mount", which remains affixed to the car. A replacement wheel with an inflated tire is then affixed to the wheel mount.

In nearly all modern passenger cars, the wheel mount will have either four or five "studs" (i.e., threaded bolts) which protrude out from the wheel mount. Those studs will fit into accommodating holes on a wheel which is carried in the car, as a spare. The use of protruding studs on a wheel mount (rather than threaded holes, recessed into the wheel mount) allows any person who is replacing the wheel to lift the new wheel and tire slightly, and place them onto the wheel mount, in a first step that does not involve any lug nuts. This makes it much easier to position a spare tire on a wheel mount, than would be required if a person had to hold a wheel and tire at an exact stationary height, while also struggling to get the end of a bolt inserted and then properly seated and started, in a recessed threaded hole.

Once the new wheel with the spare tire is in place, with all four or five studs passing through accommodating holes in the wheel, it is not good practice to screw on and then fully tighten a first lug nut, and then screw on and fully tighten a second lug nut, and then a third, and fourth, etc. Instead, each and every one of the lug nuts should progress through a "start them all, then snug them all, then tighten them all" routine, by the person replacing the flat tire.

In this context, "start" refers to getting each threaded lug nuts properly started on a threaded stud, with the threads of the nut and the stud properly engaged with each other, so that it will not damage either the nut or the stud, when the nut is forcibly screwed onto the stud.

After all four (or five) of the lug nuts have been fully and properly "started" on the studs, the next step is to get all four (or five) lug nuts properly "snugged". This term refers to a process in which the fingers (and possibly a wrench, using low force) are used to screw the nuts farther onto the studs, until a beveled or rounded surface on the inner side of each lug nut has become properly "seated" against the corresponding beveled or rounded surface of a hole in the wheel.

That "snugging" step cannot be accomplished, in a secure and reliable manner, if the operator: (i) fully tightens a first lug nut, while all of the other lug nuts remain loose; and then, (ii) fully tightens a second lug nut, while the remaining lug nuts remain loose; and then, (iii) fully tightens a third lug nut, etc.

Instead, the process of properly "seating" and securing the hub-and-tire assembly to the wheel mount is crucially important. That process can be accomplished, with much higher levels of safety and security, by "snugging" *all* of the nuts against the wheel holes, before *any* of the nuts are fully tightened.

Finally, after all lug nuts have been fully "snugged", with a modest but substantial level of tightness to ensure that the entire wheel has been properly "seated" on the wheel mount, the best way to fully tighten the lug nuts is by using a "bracketing" or "opposites" sequence. As soon as a first lug nut has been fully tightened, the next lug nut which should be tightened should be on the opposite side of the wheel (or as close to opposite as possible, if the wheel has five holes). By doing the first two tightening operations on two lug nuts which are as far apart from each other as possible, a person replacing a flat tire can make sure there is no "last second settling" or other shifting, pulling, or other motion which might raise questions about whether the new wheel is fully and properly seated on the wheel mount.

Accordingly, the entire process can be summarized as "start them all, then snug them all, then tighten them all"; or, in even shorter form, that entire sequence can be referred to as, "start, snug, tighten", so long as a listener or reader understands the full sequence.

Returning to the subject of suture anchors used during surgery, if knotless suture anchors with ratcheting mechanisms are provided and used with the types of flexible cartilage-replacing implants described herein, then those types of ratcheting anchors can enable a directly comparable "start, then snug, then tighten" installation procedure for use with such implants. Each and all of the multiple suture strands which will be used, to help securely anchor a cartilage-replacing implant to a bone, can and should go through a "start, then snug, then tighten" cycle of steps.

In this type of procedure, each and all of the suture anchors that will be used to help anchor an implant to a bone, in a load-bearing joint such as a knee, should be properly "started" (i.e., emplaced into the supporting bone) before any of the suture strands are pulled "snug", since any prematurely "snug" strand might distort or misalign a flexible implant, or otherwise render it more difficult for the surgeon to emplace all of the anchors in truly optimal positions.

After all of the anchors and strands have been "started", they should all be "snugged", in a manner which will provide gentle yet reliable assurance that the implant has become fully seated in its final desired position, with no unwanted distortions caused by any "prematurely tight" anchoring strand.

After all of the anchoring strands have been properly tensioned to a balanced and symmetric level of "snugness" around the full periphery of the implant, the final tightening steps should be carried out. This tightening step preferably should involve different suture strands in a sequence that preserves a properly balanced load distribution; in a manner comparable to the proper tightening of lug nuts on a car wheel, this can be accomplished by initially selecting and tightening suture strands positioned on opposing sides or ends of the implant.

Once that type of installing, anchoring, and reinforcing procedure is understood, and after certain types of candidate ratchet mechanisms have been explained and illustrated, then certain advantages, benefits, and improvements that can be provided the ratcheting system disclosed herein, compared to the prior ratcheting system disclosed in the two Van der Burg applications, will begin to become clear. Those advantages will be discussed below, after the mechanisms themselves have been explained and illustrated. '

This completes this introduction to the relevant prior art.

Accordingly, one object of this invention is to disclose improved designs and constructions for flexible surgical implants that are designed and suited for arthroscopic repair and replacement of meniscal cartilage, in synovial joints.

Another object of this invention is to disclose improved devices, assemblies, and methods for the stable surgical attachment and anchoring of flexible implant devices, for replacing injured or diseased meniscal cartilage, in mammalian joints.

Another object of this invention is to disclose improved devices, assemblies, and methods for anchoring flexible surgical implants designed repair and replacement of damaged meniscal cartilage.

These and other objects of the invention will become more apparent through the following summary, drawings, and detailed description.

### SUMMARY OF THE INVENTION

In view of above objects, the present invention provides an implant device according to claim 1. Advantageous embodiments may be configured according to any of the dependent claims.

Improved designs are disclosed for flexible implants that will be used to surgically replace hyaline or meniscal cartilage, in synovial (i.e., articulating) joints in humans and other mammals. In one preferred embodiment, a hydrophilic polymer, molded generally in the shape of a damaged cartilage segment that needs to be replaced, will have an enlarged peripheral rim that is substantially thicker than the interior portions of the implant. That enlarged peripheral rim will be designed to fit, in an accommodating manner, into a "groove" that will be machined (with the help of templates, computerized tools, etc.) into the bone surface that will receive and support the implant. This will create an interlocking-type "fit" (or seating, or similar terms) that will provide greater strength and stability for the implant, to allow it to resist compressive, lateral, shearing, and other forces and stresses that will be imposed on the joint and the implant, even during falls, accidents, or other moments of peak loading or stress.

For maximal strength, reliability, and durability, an anchoring component that can withstand high tensile stresses should be embedded within the enlarged peripheral rim of the implant. If desired, this component can be a solid "stabilizing ring", made of a shape-memory and/or super-elastic material, such as a "nitinol"-type alloy. Alternately (and preferably, to avoid concerns about possible long-term corrosion), the embedded anchoring component can be made from a multi-stranded flexible wire, cable, or similar component, made of braided or twisted strands, to give the anchoring component a non-smooth surface that will help prevent any slippage, creep, or other unwanted motion despite multiple years of use.

Placement of a flexible cable component, within the molded polymeric rim of an implant, can allow the implant to be flexed into a cylindrical or other elongated, compressed, or similar shape, for minimally-invasive insertion into a joint (such as via an arthroscopic insertion tube). After the implant has entered the joint, it will emerge from the insertion tube, or otherwise will be allowed or caused to return to its normal shape. This will enable the implant, with its enlarged outer rim containing the flexible cable or stabilizing ring component, to perform a reinforcing and stabilizing role when the enlarged and reinforced flexible polymer rim of the implant settles into the bone groove.

Various types of anchoring means, such as "snap caps" that will attach to the rounded heads of bone screws, can be coupled to the flexible cable or stabilizing ring component, at spaced locations around the outer rim of the implant device. Alternately or additionally, devices such as suture strands, cerclage wires, reinforcing mesh extensions, or other devices or materials having "free ends" that can be secured to bone surfaces or soft tissue, can be coupled to the flexible cable or stabilizing ring component, at spaced locations around the outer rim of the implant device. If suture strands are used, they can pass through certain types of "ratcheting knotless suture anchors", as described herein, which can provide a surgeon with enhanced means for anchoring an implant in its targeted location in a strong, secure, and reliable manner.

### BRIEF DESCRIPTION OF THE DRAWINGS

FIGURE 1 is a perspective view of the upper (or exposed, or articulating) surface of a surgical implant for replacing hyaline cartilage, showing a flexible polymer component with a smooth and lubricious articulating surface, and also partially showing anchoring screws on the underside (or anchoring surface) of the implant.
FIGURE 2 is perspective cutaway view of the surgical implant, showing a flexible stabilizing ring (which can be made of a shape-memory or super-elastic material) that is embedded within an enlarged peripheral rim made of a flexible synthetic polymer. Screw-holder caps (which will snap onto the heads of anchoring screws, after the screws have been emplaced in a supporting bone surface) are affixed to the stabilizing ring, at spaced locations around the periphery of the implant.
FIGURE 3 is a perspective view of the anchoring surface (or underside, or similar terms) of the surgical implant shown in FIG. 2.
FIGURE 4 is a perspective view of an anchoring screw, with a rounded "snap cap" head, and with a "shoulder ring" affixed to the neck of the screw, which will press against a stabilizing washer that will press against a bone surface.
FIGURE 5 is a perspective view of a screw-holder cap, affixed to a stabilizing ring of an implant, which has been "snapped" onto the rounded head of an anchoring screw.
FIGURE 6 is a cutaway side view depicting an anchoring component, in the shape of a large "washer" with an open center, which is securely affixed to a supporting bone by means of screws, pins, cement, etc. After that "anchoring washer" has been affixed to a bone, a flexible polymer implant is inserted into the joint, and the enlarged rim of the implant is pressed and nestled into a groove or trench that has been machined into the supporting bone surface. A circular ring, affixed to the anchoring washer, will cause the flexible polymer implant to "snap" into the groove or trench in the bone surface, and will thereafter prevent dislodgement of the flexible implant For simplicity, FIG. 6 illustrates only the enlarged peripheral anchoring rim 112 of the cartilage-replacing implant; the central polymer component of the implant is not shown.
FIGURE 6 ALSO indicates that the enlarged rim 112 of the implant contains a reinforcing component 141 with a non-flat arc-shaped cross-section, which emulates a metal tape measure. That cross-sectional shape enables the reinforcing component to be squeezed until it undergoes a "collapsible transition", which will allow it to be inserted into a joint in compacted form. Subsequently, when the implant and rim return to their unstressed manufactured shape, reinforcing component 141 will return to its arc-shaped cross-section, and will become stiff and strong again.
FIGURE 7 is a perspective view with a partial cutaway section of a flexible implant with an anchoring cable embedded within its outer rim. Rather than using snap caps that will attach to the heads of anchoring screws, this implant uses anchoring pegs with "sawtooth" surfaces that will engage anchoring sleeves, which can be emplaced in a bone surface before the implant is inserted into a joint that is being repaired.
FIGURE 8 depicts: (i) an anchoring cable which will be embedded within a surgical implant that is sized and designed to replace the cartilage of a femoral runner, in a knee joint; and, (ii) four braided suture segments which have their center portions wrapped around the anchoring cable at spaced locations, thereby generating suture segments having free ends which pass through ratcheting knotless suture anchors designed to be embedded in hard bone.
FIGURE 9 depicts the same anchoring cable and suture segments shown in FIG. 8, where the anchoring cable is embedded within a polymeric hydrogel component, while the free ends of the suture segments emerge from the surface of the polymer component.
FIGURE 10 (which is prior art) depicts a "classic" ratchet system. Its rotating gear is constrained by a spring-mounted pawl, in a manner which allows the gear to rotate in only one direction.
FIGURE 11 (which is prior art) depicts the type of cam cleat that is used to secure a rope on a sailboat.

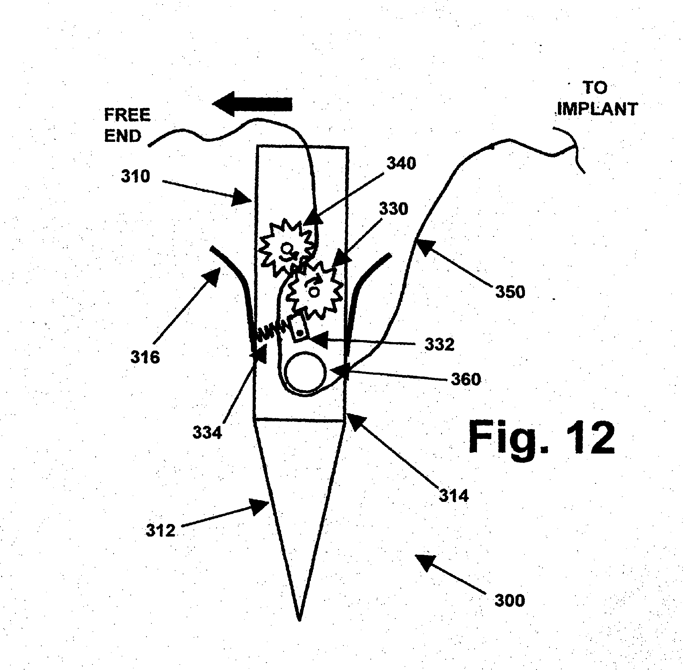

FIGURE 12 is a cutaway view showing the mechanism inside a miniaturized double-gear ratchet system within a knotless suture anchor, which will allow a surgeon to adjust, in a stepwise manner, the tension on an anchoring and/or reinforcing suture strand.

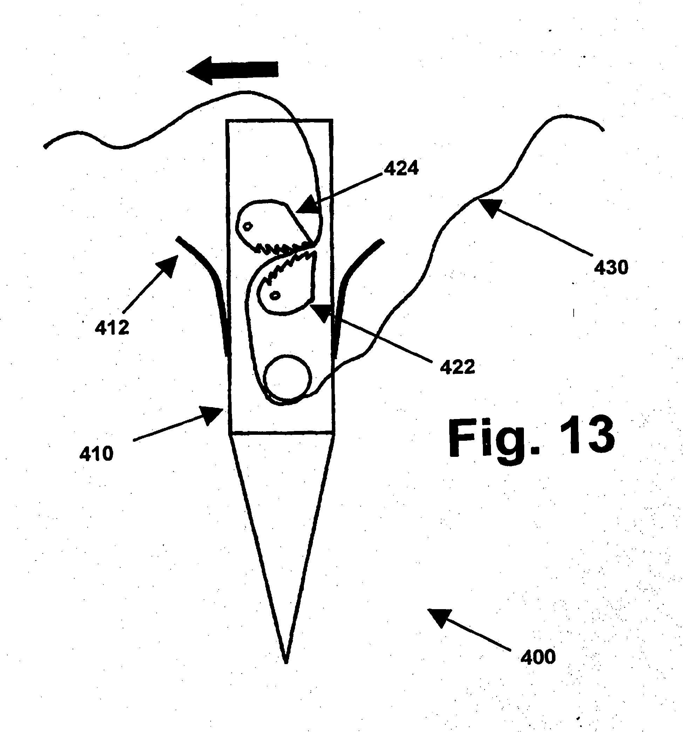

FIGURE 13 is a cutaway view showing the ratcheting mechanism inside a miniaturized knotless suture anchor, containing two axle-mounted non-circular (cam-type) gripping components which will act in a manner comparable to the cam-cleats that are used to adjust and tension ropes on sailboats.

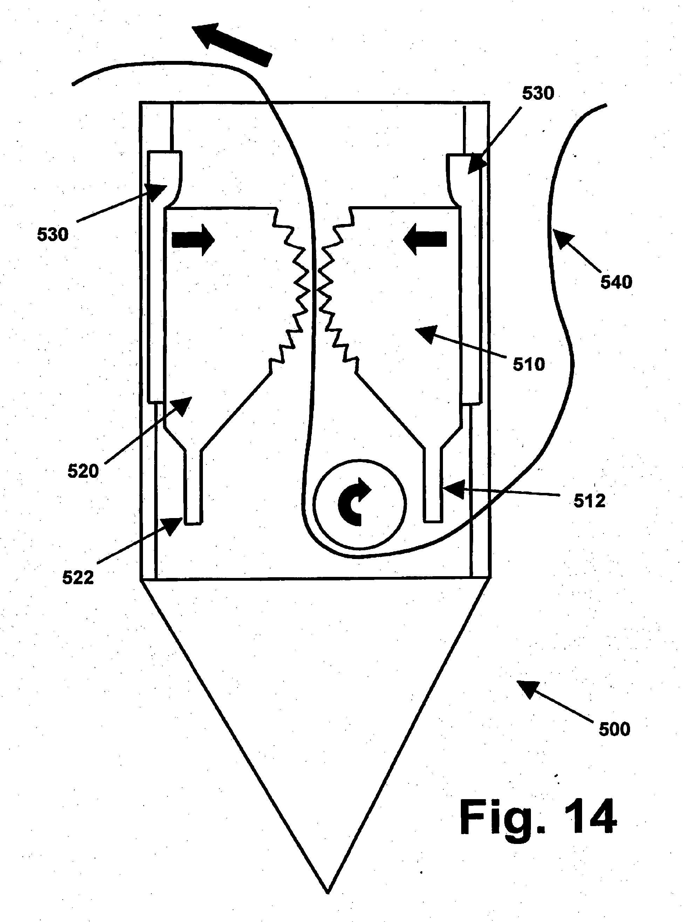

FIGURE 14 is a cutaway view showing a ratcheting cam-cleat mechanism in which the two cam-shaped gears are mounted on flaps or extensions, rather than axles, and are pressed inwardly by a surrounding sleeve made of an elastomeric polymer.

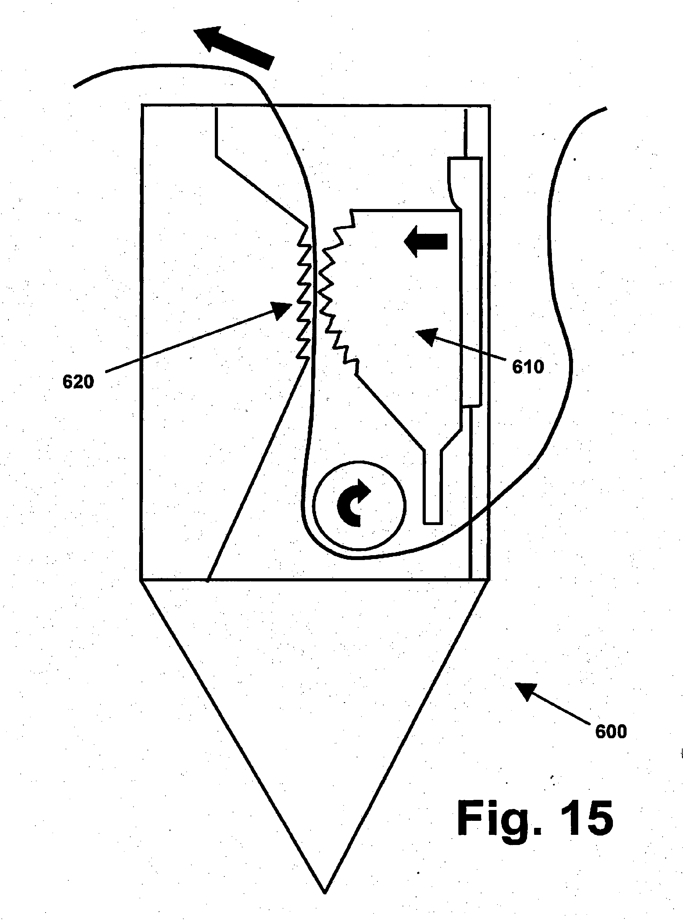

FIGURE 15 is a cutaway view of a knotless suture anchor containing a single axle-mounted ratcheting component, which will interact with a fixed sawtooth surface on an interior wall of the suture anchor to establish ratcheting control over a braided suture strand that passes through the suture anchor

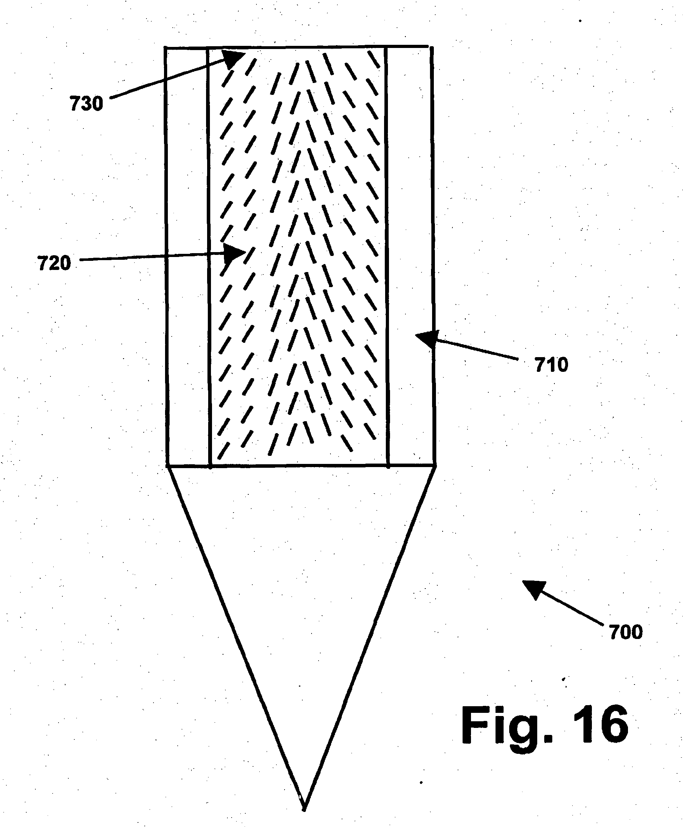

FIGURE 16 is a cutaway view of cylindrical sleeve device with numerous stiff bristles lining the internal surface of the sleeve, angled toward the outlet end of the sleeve. A braided suture strand can be pulled through the sleeve toward the outlet end, with minimal resistance. However, if a tensile force attempts to pull the suture strand in the opposite direction, bristles will become lodged in the interwoven surface of the braided strand, and will prevent the strand from moving in the non-allowed direction.

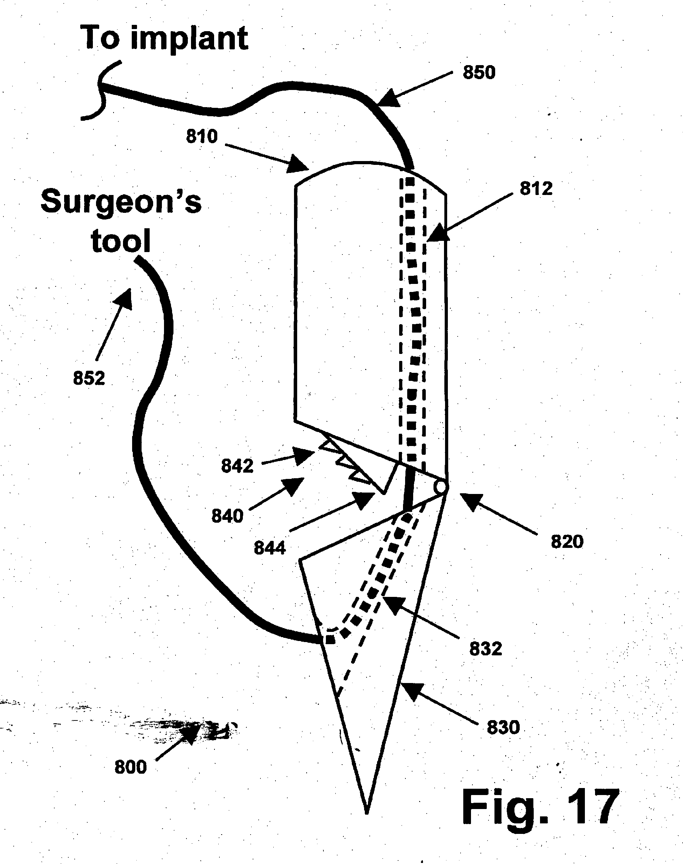

FIGURE 17 depicts an anchoring suture which passes through a cylindrical anchoring component that is designed to flex in a manner which will effectively crimp the suture strand, in a manner which will secure it to the anchoring device, wherein partial crimping of the device will provide a ratcheting mechanism that can help a surgeon properly emplace and "seat" a cartilage-replacing implant.

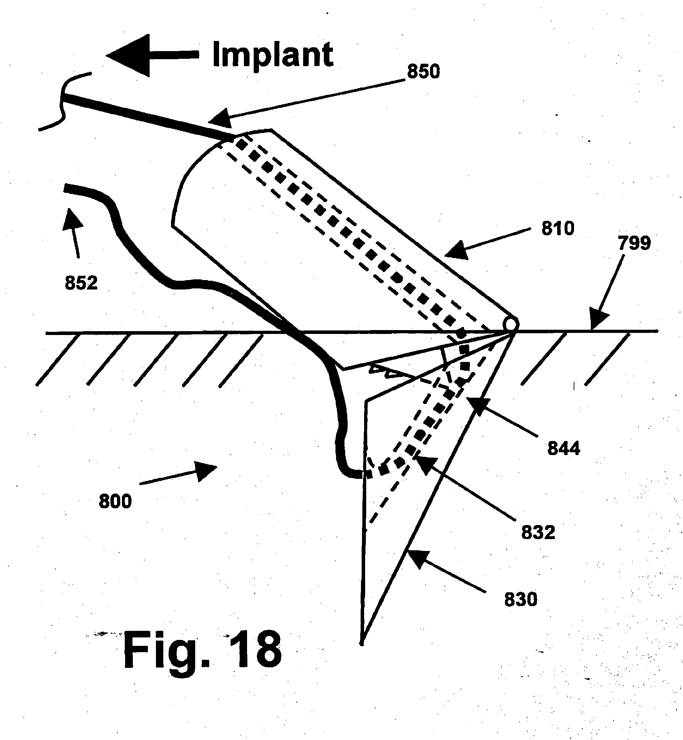

FIGURE 18 depicts an anchoring suture passing through the same crimping anchor shown in FIG. 17, after the crimping anchor has been driven into a bone surface.

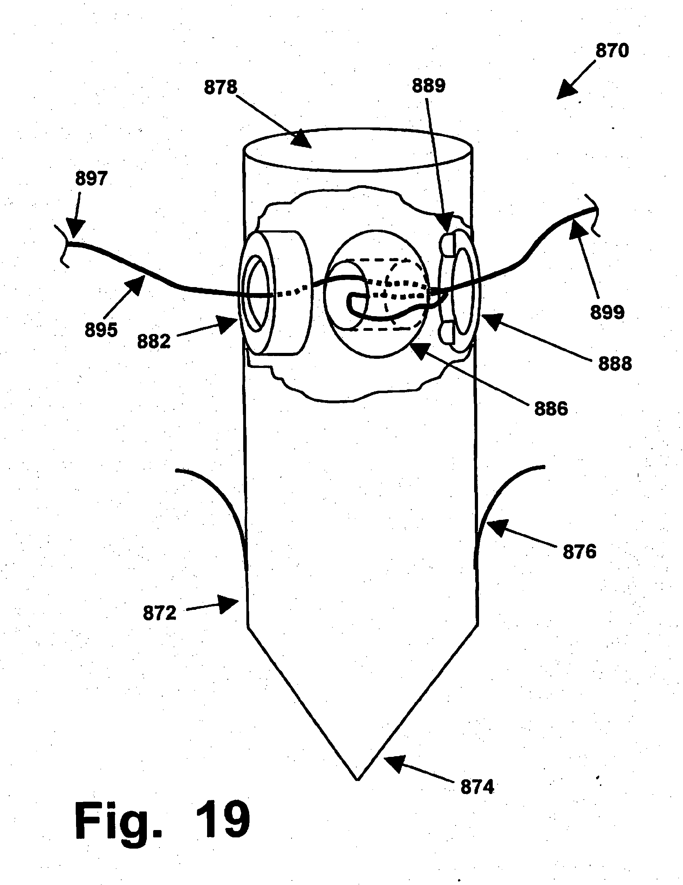

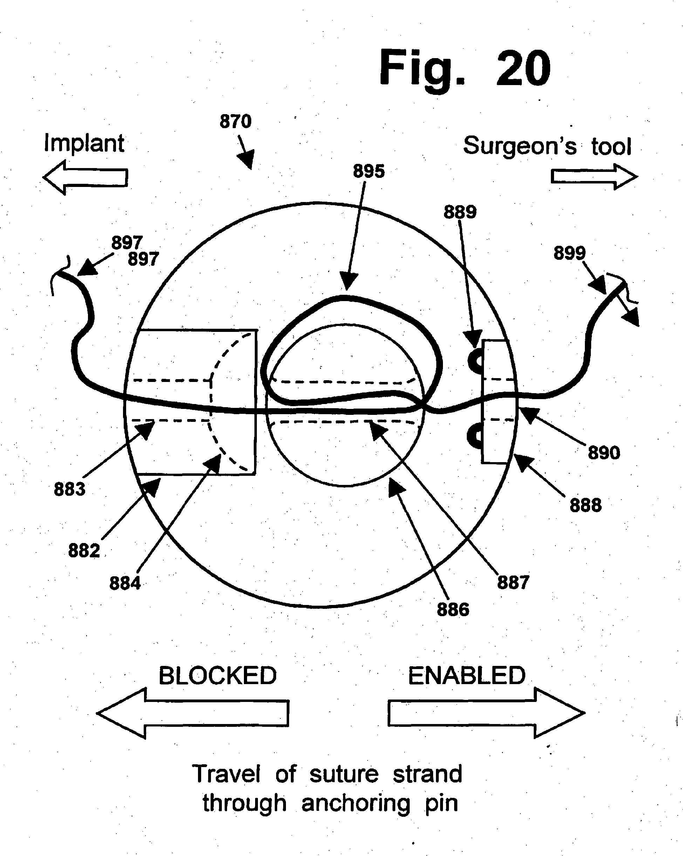

FIGURE 19 is a side (elevation) view, with a partial cutaway, showing another type of ratcheting knotless suture anchor (also shown in FIG. 20) having a movable "brake" (or bead, etc.) with a suture strand wrapped around it. When a surgeon pulls the free end of the suture strand (i.e., from the right side of device shown in FIGS. 19 and 20), the suture strand will be pulled through the bead, with a sliding motion. When the surgeon releases the suture strand, the tension exerted on it by the implant device will pull the movable brake/bead against a "seating component" having a conforming shape. This will pinch and effectively trap the suture strand, to prevent it from being pulled toward the implant.
FIGURE 20 is a top (plan) view of the ratcheting suture anchor shown in FIG. 19.

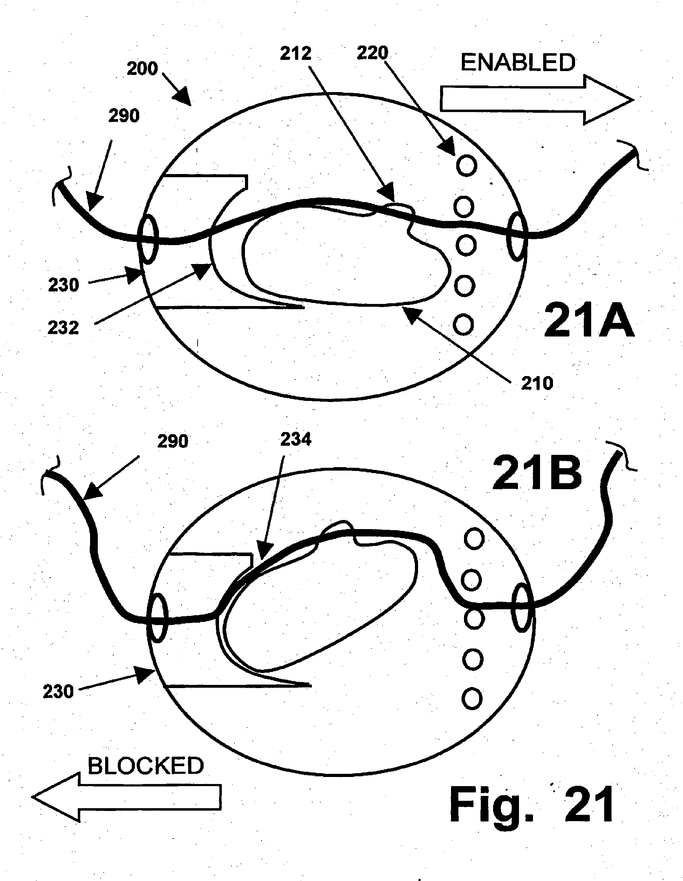

FIGURE 21 (which includes panels 21 A and 21 B) is a cutaway elevation view of a ratcheting knotless suture anchor having a movable "brake" (or bead, or similar terms) which is non-spherical, and which has a side-mounted coupling device thatg allows a suture strand to pass through it.

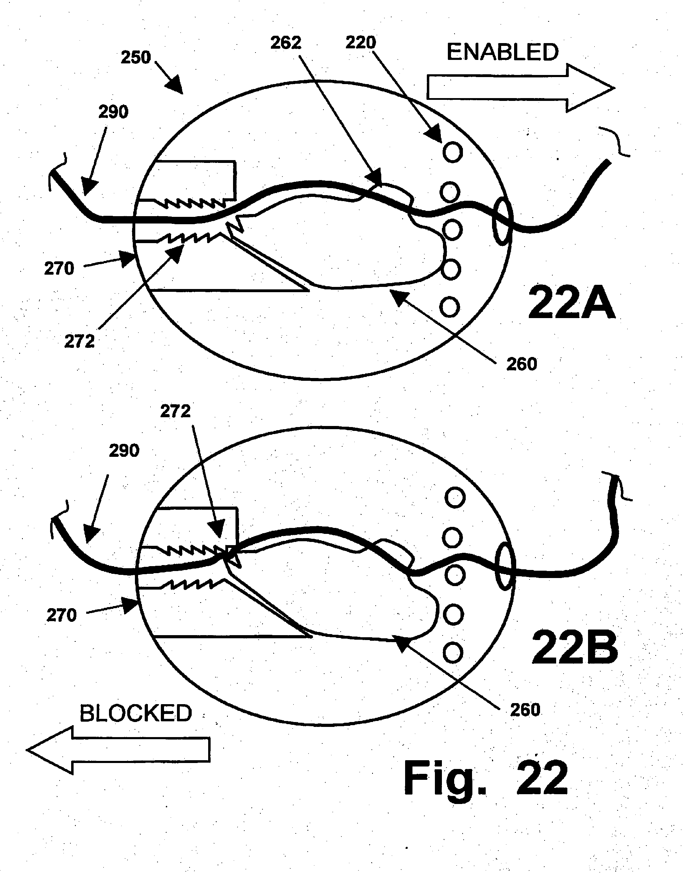

FIGURE 22 (which includes panels 22A and 22B) is a cutaway elevation view of a ratcheting knotless suture anchor having a movable brake (or bead, or similar terms) which is non-spherical, with a side-mounted suture-coupling device, and with prongs, pins, and/or ridges on both the break and the seating component that will press into and grip a braided suture strand if a tensile force pulls the movable brake into engagement with the seating component.

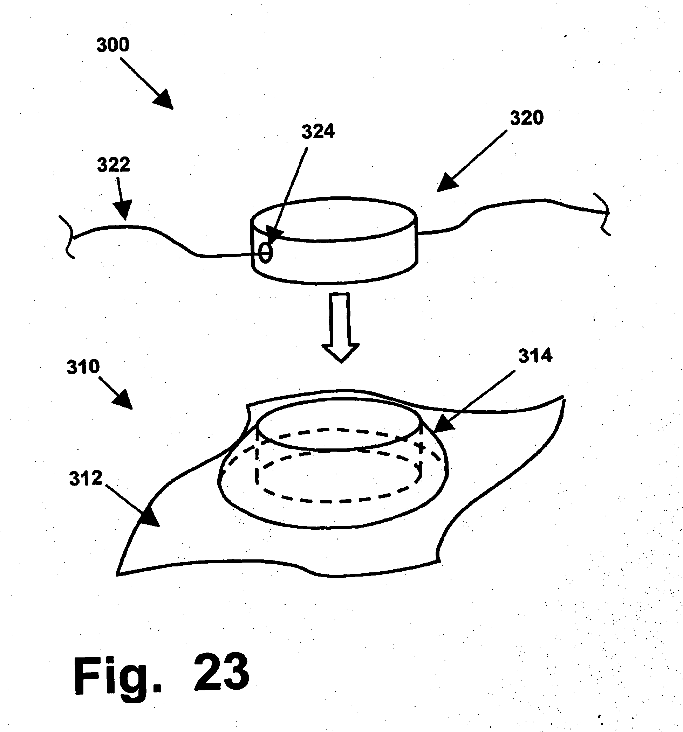

FIGURE 23 depicts a ratcheting knotless suture anchor which is made by coupling together: (i) an anchoring device, shown with a flexible skirt component that can be affixed to soft tissue, such as by sutures or staples; and, (ii) a racheting subassembly having a braided suture strand passing through it.

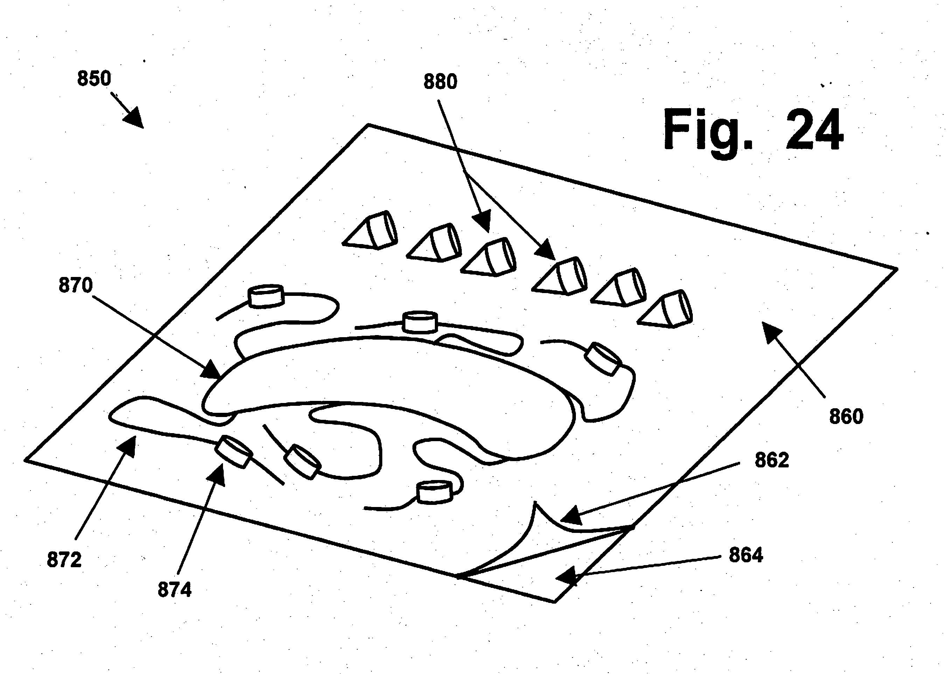

FIGURE 24 depicts a surgical kit, containing an implant device and a plurality of ratcheting knotless suture anchors, within a sealed envelope which keeps those components sterile until the kit is opened during a surgical operation.

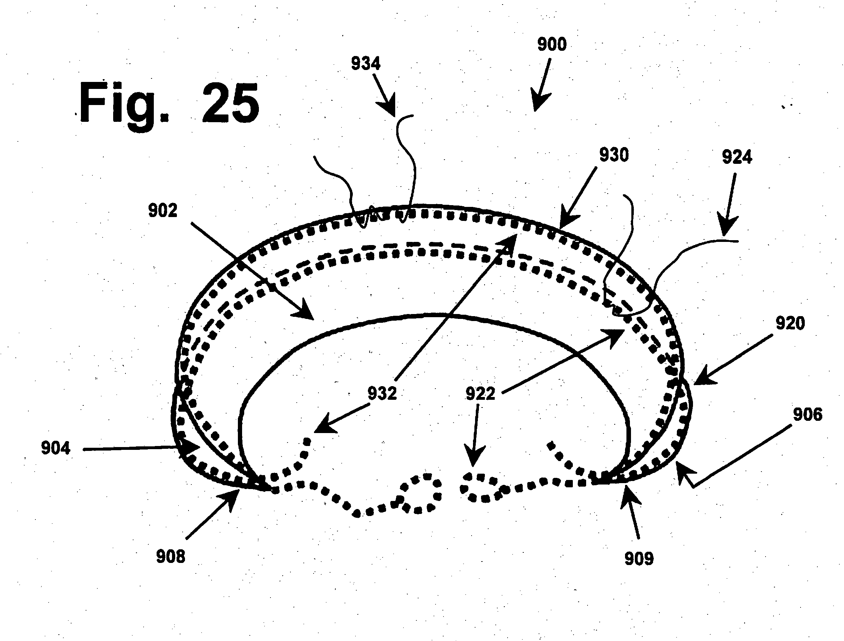

FIGURE 25 is a cutaway view, showing upper and lower flexible anchoring cables embedded within a polymer segment having the size and shape of a meniscal wedge. Two suture strands are shown wrapped around the cables, with both ends of each suture strand emerging from the polymer segment; this allows the ends of the suture strands to be used to anchor the peripheral surface of the meniscal implant, to soft tissues that form the "capsule" which surrounds a knee and holds in the synovial fluid. The ends of the anchoring cables are designed to be affixed to the tibial plateau, in a manner that emulates the anchoring of natural meniscal segments.

### DETAILED DESCRIPTION

As briefly summarized above, a surgical implant 100, designed for replacing a relatively large segment of hyaline cartilage in a synovial joint such as a knee, shoulder, hip, etc.) is illustrated in FIGS. 1-3. Scaled-down implants with the same structures described herein, but with smaller diameters and thicknesses (and with only one or two anchoring screws, pins, or other components, which also can be smaller) also can be created for replacing hyaline cartilage in smaller joints, such as in thumbs, fingers, wrists, etc.

For simplicity of illustration, implant 100 is shown as having a generally round and flat shape. In actual use, any such implant designed for a large joint should have a molded and shaped articulating surface that will closely emulate the size and shape of the cartilage segment that is being replaced by the implant. The range of sizes and shapes of such cartilage surfaces (which vary widely, for sifferent joints, and which also are affected by gender, age, body size and weight, and certain other factors) are well-known to orthopedic surgeons. Such implants can be manufactured in an assortment of sizes and shapes, and a surgeon who is repairing a joint will select one or more implants having optimal sizes and shapes for a specific patient, based on X-rays or similar images or measurements of the joint that needs to be repaired in that patient.

On that subject, it should be noted that the irregular surface of a diseased, injured, or otherwise damaged or defective cartilage segment in any load-bearing joint typically will abrade and damage any other cartilage segment(s) that rub against the damaged and irregular surface; therefore, most such repairs will require at least two implant devices. For example, if a femoral runner needs to be replaced, then the portion of the tibial plateau which rubs and slides against the damaged femoral runner will likely also need to be replaced. This is conventional practice in this type of orthopedic surgery, and the implant devices described herein will be well-suited for such use, if manufactured in a range and assortment of sizes and shapes that will allow a surgeon to select suitably-sized implants based on the size, weight, and needs of any specific patient.

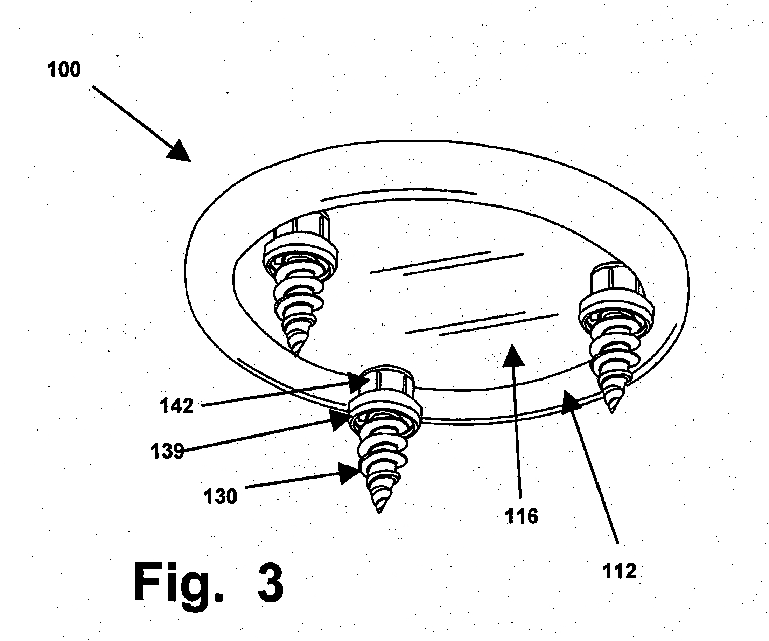

Returning to FIGURES 1-3, the polymer component 110 of an implant 100 preferably should be made of a single molded component to avoid and eliminate any surface seams (which can also be referred to as junctures, junctions, fissures, etc.) which otherwise might become potential weak spots, focal points for stress, and/or sites or sources of abrasion. Polymer component 110 comprises an enlarged peripheral rim 112 (shown in cross-section in FIG. 2), a smooth and lubricious articulating surface 114 (which will be coated and lubricated by natural synovial fluid, after implantation into a mammalian joint), and an underside or anchoring surface 116 (shown in FIG. 3). Any directional terms used herein (such as up, down, top, bottom, above, under, etc.) assume that a bone surface provides a horizontal "floor" (or base, support, foundation, etc.) for an implant, and the implant will rest on top of that horizontal base, with the anchoring surface (underside) of the implant resting upon the supporting bone surface, and with the articulating surface (which might can be called the "exposed" side of the implant) facing upward, on the "top" surface of the bone.

The types of polymers of interest herein can be manufactured by any of several molding methods that are known to those skilled in the art, using a flexible but tough and durable hydrophilic polymer, such as a suitable hydrophilic polyacrylonitrile (PAN) or polyurethane. As known to those skilled in polymer chemistry, terms such as polyacrylonitrile and polyurethane refer to the types of chemical linkages that are used to create the long "backbone" chains within such polymer molecules. Any of numerous types or combinations of "side groups" (also called moieties, pendant groups, and various other terms) and/or reactive crosslinking groups can be chemically bonded to the backbone chains. This is usually done by proper selection of the "monomer" reagents that are used to create a polymer; when monomer "links" are bonded to each other to form the long "backbone" chains in a polymer, the side groups that were present in the monomers will become pendant groups attached to the long backbone chains of the polymer.

It also is well-known and conventional to mix together different types of monomer reagents, when forming polymers. For example, if 10% of the monomer reagents in a mixture have cross-linking moieties as part of their side (pendant) groups, the resulting polymers will have cross-linking groups that will be positioned, on average, 10 "units" apart, along the lengths of the backbone chains. Similarly, if 1% of the monomer reagents in a mixture are "chain-terminating" compounds, the resulting polymeric chain lengths will average out to be roughly 100 units each, in the completed polymeric material.

Accordingly, a polymer molecule which falls within.a certain category or label (such as polyacrylonitrile, polyurethane, etc., as determined by the types of linkages in the long "backbone" chains) can be created with nearly any desired types of side (pendant) groups, which will impart a set of desired traits to the final polymer. A combination of factors, which will depend mainly upon: (i) the selected side groups that have been incorporated into the polymeric molecules, via monomer selection; (ii) the lengths of the polymeric backbone chains; and, (iii) any mixture of two or more different types of polymeric molecules that are included in the final preparation, will control whether a polyacrylonitrile, polyurethane, or similar polymer will be hydrophobic or hydrophilic, flexible or rigid, permeable or impermeable to water, etc.

If desired, the exposed articulating surface of an implant of this type can be given a controllable negative electrical (ionic) charge, by means such as contacting the articulating surface for a controlled period of time with dilute sulfuric acid, as described in published US patent application 11/105677, entitled, "Hydrogel implants for replacing hyaline cartilage, with charged surfaces and improved anchoring". This type of treatment can create a polymer surface that closely emulates the negative charge density of natural cartilage, which in turn can improve the interactions between the implant surface, and certain types of positively-charged components of synovial fluid.

The entire polymer component 110 (or any portion thereof) of an implant 100 can be reinforced by an embedded flexible fiber mesh, if desired, so long as the fiber mesh is not exposed on articulating surface 114, which must be extremely smooth and slippery. If desired, a portion of any such embedded mesh can extend outside of the peripheral rim 112, to provide additional anchoring means. Alternately or additionally, strands of anchoring material (such as suture strands, metal wires, flat polymeric or metal eyelets, etc.) and/or one or more sheets or segments of flexible mesh or drape material, also can be affixed to the implant (such as at or near the anchoring screws), either during the manufacturing process, or by a surgeon immediately before or during implantation, to provide additional anchoring strength and stability.

It should be noted that placing a "non-homogenous" member inside a polymer component can sometimes weaken the overall strength of the polymer component. Nevertheless, the types of reinforcing members disclosed herein can play highly valuable roles in achieving and providing truly stable and durable anchoring systems. Accordingly, any references to "reinforcing" components (or related phrases), as used herein, are used to refer to embedded or attached components that can lead to either or both of the following results or effects:
(i) a stronger component or assembly, such as a complete implant assembly having a molded polymer component that is able to withstand higher compressive, shearing, or other stresses and loads; and/or,
(ii) a stronger, more secure, and more durable anchoring attachment to a hard bone surface or other tissue.

For simplicity of illustration, the "underside" (or "anchoring surface") 116 of implant 100 is depicted as a smooth surface, in FIG. 3. In an actual implant, the anchoring surface (which normally will contact and press against a prepared bone surface, from which damaged native cartilage has been removed) preferably should have a fibrous, porous, or similar texture that will actively encourage the ingrowth of scar and/or bone tissue, to provide stronger and more stable anchoring of the implant to a supporting bone surface (or other type of tissue, such as in the case of meniscal or labral implants). There are several known ways to create, in molded polymers, the type of porosity that will promote cellular ingrowth. Alternately, the underside 115 of polymer component 110 can be bonded to an additional layer of ingrowth-and-anchoring material, such as a screen or mesh layer made of several layers of very thin and flexible wires made of a titanium or other biocompatible alloy. In addition, any such anchoring surface can be coated or impregnated with one or more hormones or growth factors that will accelerate the ingrowth of tissue into the anchoring surface of the implant, to promote faster recovery after surgery.

In a preferred example, a stabilizing ring 140 is embedded within the flexible polymeric component of peripheral rim 112. Ring 140 can also be referred to by various other terms, such as an anchoring component or anchoring ring, or as a cable ring, cable anchor, anchor cable, or similar terms if it is made of a cable-type material. The term "cable" as used herein implies an elongated flexible component made from a multi-stranded material, which in most cases will be a flexible metal, or a synthetic polymeric material, such as "ultra-high molecular weight polyethylene" (UHMWPE).

If desired, ring 140 can be made of a "shape-memory" or "super-elastic" material. Because a component which is fully embedded within a polymer component will not be contacted by body fluids in any appreciable quantity, a "nitinol"-type alloy can be used for this type of embedded component, if desired, despite the risks of gradual corrosion that can occur over a span of years or decades when nitinol alloys are contacted by body fluids. Alternately, to alleviate potential regulatory concerns, and to render safety- and durability-testing easier, a shape-memory, super-elastic, or other polymer can be used to make stabilizing ring 140; or, stabilizing ring 140 can alternately be made of a twisted or braided multi-strand cable, as described below and illustrated in FIG. 7.

FIGS. 8 and 9 were both introduced (and their callout numbers were identified) in the Background section. Briefly, FIG. 8 depicts a flexible anchoring ring 212 (which can be made of shape-memory or super-elastic material, or from a multi-strand cable), with a plurality of anchoring sutures 214 and 216 wrapped around it at spaced locations, and with ratcheting knotless suture anchors 220 and 222 affixed to the anchoring sutures. FIG. 9 depicts a flexible implant device 200, having the size and shape of a femoral runner in a knee joint, which contains the flexible anchoring ring 212 of FIG. 8 embedded within the outer periphery or rim of a molded polymer component 230. One additional comment that merits note is that the anchoring sutures can be color coded or otherwise marked (such as with varying numbers of solid bands) to provide a surgeon with an additional set of visual cues that can help the surgeon complete an installation quickly and effectively.

The type of implant assembly shown in FIGS. 1-9, containing a flexible stabilizing ring 140 embedded within an enlarged flexible polymeric component around the peripheral outer rim 112 of an implant device, can allow a flexible surgical implant of this type to be bent, rolled, or otherwise flexed (without requiring tools, and with tolerable stresses that will not cause any lasting damage to the implant) into a cylindrical or compressed shape, for insertion into a joint via an arthroscopic insertion tube.

If the shape-memory and/or super-elastic stabilizing ring has a temperature-dependent behavior that causes it to become more flexible and less rigid when it is chilled, then it can be chilled, immediately before flexion and insertion, by a suitable step such as immersing it in a bowl of ice-cold saline slush. As the implant warms back up to body temperature, after it is inside the joint, the shape-memory stabilizing ring will stiffen, imparting a reinforced final shape to the implant.

Alternately, if the stabilizing ring is made of a super-elastic material that does not require temperature manipulation to give it high levels of flexibility, then no such treatments are required, and other approaches can be considered. For example, if an elongated component made of a super-elastic material has a half-circle or arc-shaped cross-section, similar to the cross-sectional shape of a tape measure, as depicted in the stabilizing ring shown in FIG. 6, then it can offer substantial resistance to bending, but only up to a certain point (or level, extent, or similar terms) of flexion. When that point or level is reached, the arc-shaped cross-section will be forced and flattened into a linear cross-section, in a manner comparable to what happens to a metallic tape measure when it is retracted into a carrying case. After that transitional deformation and flattening occurs, at one or more locations around the rim of an implant that is being flexed and rolled up, the stabilizing ring component can be bent with almost no resistance, thereby allowing an implant with this type of stabilizing ring to be inserted into a joint via an arthroscopic insertion tube. Once the implant is inside the joint, it is allowed to relax and return to its manufactured size. When that occurs, the stabilizing ring will regain its original cross-sectional arc shape, and when that occurs, it will become much stiffer, in a way that will render it ideally suited for reinforcing, stiffening, and strengthening a cartilage-replacing implant.

Regardless of which specific approach is used, a stabilizing ring made of a shape-memory or super-elastic material can perform its reinforcing role, once the rim of an implant with that type of embedded ring has been properly positioned in a groove that has been machined (by the surgeon, with the help of templates, computerized tool guides, etc.) into the bone surface that will support the implant.

In a preferred example, stabilizing ring 140 can be provided with means for securing the implant to anchoring components, such as bone screws. Alternately or additionally, the periphery of the implant can be provided with other anchoring means, either directly or indirectly, which can be secured to bone or soft tissue by various known means, including staples, sutures, screws, etc.

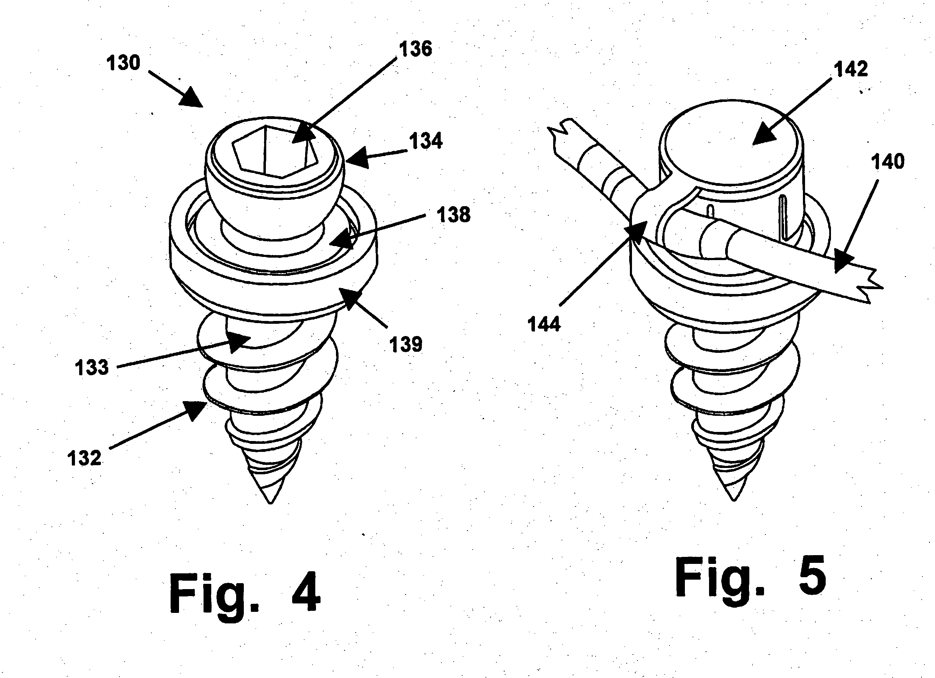

In the simplified embodiment shown in FIGS. 1-3, three anchoring screws 130 will be emplaced in the supporting bone, before the implant is inserted into the joint. This can be done with the aid of pilot holes that will be drilled into a prepared bone surface (from which the native cartilage has been removed), using a template or computerized guiding tool to establish the proper locations and angles of the screw holes and screws. As shown in FIG. 4, the threads 132 of each bone screw 130 (on shaft 133) will have sizing and spacing suited for bone anchoring, and each screw head 134 will have a rounded outer shape, to allow a "snap cap" 142 (affixed to stabilizing ring 140) to be secured to a screw, by simply pressing snap cap 142 onto a screw head 134. Torsional driving means (such as a hex socket 136, as shown in Fig. 4) should be provided on each screw head 134, to enable the surgeon to drive each screw 130 into the bone, to a desired depth.

If desired, an enlarged "shoulder ring" 138 (or washer, or similar terms) can be provided around the "neck" of each bone screw 130, as shown in FIG. 4. The shoulder ring 138 can press directly against a bone surface, if desired; alternately, it can settle into an accommodating washer component 139. If a washer component 139 is used, the bottom surface of shoulder ring 138 preferably should have a beveled, angled, or rounded surface, rather than a completely flat and planar disc-type rim, and the "seating surface" inside washer component 139 should have an accommodating beveled or rounded surface. This will enable more stable and secure seating of the screw 130 in washer component 139, if a slight misalignment occurs where a screw hole drilled into a bone surface is not exactly perpendicular to the bone surface at that location.

As shown in greater detail in FIG. 5, each "snap cap" 142 can be secured to stabilizing ring 140, by any suitable means, such as a protruding tab (or finger, strap, or similar terms) that can be bent into a loop structure 144 that will encircle ring 140. At each of the spaced attachment locations around the length of stabilizing ring 140, a "coupling detente" 146 can be provided. In this context, this type of "coupling detent" can refer to a localized bend, a drilled hole, a welded or crimped component, or any other device or component that will prevent sliding, slippage, or other displacement of the loop structures 144 (or similar components) along the length of an anchoring rim 140, when those componentsare embedded within a polymer component.

In one preferred example, coupling detentes 146 can consist of a "bend" that places the actual coupling location (i.e., the site where loop 144, on a "snap cap" 142, wraps around anchoring rim 140) closer to the surface of the bone that will support the implant. Coupling detentes that have this arrangement can provide anchoring components that are partially or fully "countersunk", in a manner that allows the top of a screw head, "snap cap", or other anchoring structure to be "lower" (i.e., closer to and possibly aligned or "flush" with the bone surface), with less protrusion. This arrangement can reduce the risk that a protrusion (or bump, hump, etc.) at the site of an anchoring component might either (i) damage the flexible polymer component of an implant, or (ii) create an unwanted irregularity in an otherwise flat or smoothly-rounded articulating surface, after an implant has been installed.

If desired, stabilizing ring 140 can be provided with a closure sleeve 149 or 213 (as illustrated in FIGS. 2 and 8), to hold the two ends of a stabilizing ring 140 together. If used, this type of closure sleeve 149 can be secured to the two ends of a strand, cable, or other components, by means such as crimping, a rivet, "snap rings" inside the sleeve 149, or similar means. In general, shape-memory and super-elastic materials are not well-suited for welding, and the types of stresses imposed on them often focus on any junctures or interfaces, in ways that often render glue or epoxy unreliable, and prone to failure. Therefore, other means of securing the two ends of a stabilizing ring, to each other, must be used, such as a crimped closure sleeve that tightly grips both ends of a ring.

Another configuration that merits evaluation can use a "key-ring" arrangement, in which the two ends of a stabilizing ring 140 overlap each other for some distance. Since both of the two ends will be embedded within a tough and durable polymer (if desired, a metallic or other sleeve, wire, or tape can be tightly wrapped and/or crimped around at least a portion of any such overlap), this approach is likely to be useful in at least some designs, especially non-circular designs. Any such juncture preferably should be positioned, within any stabilizing ring in an implant as disclosed herein, in a location that will not be subjected to high flexure-related stresses, during the insertion stage of the operation. For example, if a femoral runner implant has a shape comparable to a ellipse or an oval-type racetrack, the juncture location preferably should be positioned near the middle of the most nearly straight portion of the ring, rather than near the "apex" of a curved portion of the ring.

Alternately, it is feasible and practical to provide a gap between the two ends of a stabilizer ring, if desired. Since the nature and purpose of the ring is simply to provide stabilization for the implant after the enlarged peripheral rim of an implant has settled into an accommodating groove or trench that has been machined into the surface of the supporting bone, there is no specific need for a stabilizer ring or anchoring cable to extend around the entire peripheral rim of an implant. For example, each femoral runner implant can be provided with an enlarged peripheral rim made of molded polymer material, which will contain embedded stabilizer segments mainly located around the "curved ends" of the implant, while the two "side" portions (medial and lateral) of the implant periphery might contain stabilizer segments that are long enough to provide secure anchoring attachments at or near all of the ends of the segments, but which do not comprise a complete "ring" that fully encircles and surrounds the implant.

The molding and fabrication methods that will be required to make these types of implants are well within the level of ordinary skill in that field of art. Any of various mechanical means can be used to suspend a stabilizing ring at an appropriate height and position in a mold cavity, while a liquid "pre-polymer" is poured into the mold cavity, so that the stabilizing ring will be properly centered and embedded within the enlarged peripheral rim of the implant after the "pre-polymer" mixture has set (or cured, hardened, polymerized, etc.) to form the flexible polymer. For example, if the stabilizing ring of an implant has "snap caps" affixed to it, which are designed to be snapped onto the rounded heads of anchoring screws that have been emplaced in a supporting bone, then the molding cavity can include (or interact with) a device (often called a "jig") that will have the same number of rounded screw heads, positioned in the same spatial relationship with respect to the enlarged rim vacancy in the molding cavity.

Optimal designs for different types and sizes of implants, for different types of joints and among different classes of patients, are likely to vary substantially. For example, finger and thumb joints are small, and do not need to withstand nearly the loadings and stresses that are imposed on knee joints; accordingly, implants as disclosed herein for repairing finger or thumb joints can rely entirely on suture strands that are wrapped around a peripheral anchoring cable, and that emerge from the outer rim of the molded polymer component. By contrast, in most patients, implants for repairing a femoral runner or tibial implant, in a knee joint, will need to withstand much greater loads and stresses. Accordingly, any implants used for knee repairs normally should utilize a combination of bone screws and suture strands; however, even that presumption will need to be assessed, for each individual patient, by a skilled orthopedic surgeon, depending on the status and needs of the patient. For example, if a surgeon is treating an elderly woman who is suffering from serious osteoporosis and/or brittle bones, the surgeon might decide that bone screws would pose an unacceptable risk of damaging that patient's already-fragile bones, so other anchoring means should be used instead of bone screws.

Accordingly, when such factors are taken into account, the design options that should be considered for specific implants in specific patients become broader, and the following factors should be taken into account.

For implants that will remain under relatively steady or low-level compressive loadings that do not need to withstand high shear stresses, such as in finger or thumb joints, relatively aggressive anchoring components such as screws may not be required. Devices such as staples, sutures, and/or pins made of swellable materials (or using "spring-type" gripping mechanisms) can provide adequate alternatives for at least some such implants.

In addition, depending on the depth and shape of a groove or trench that will be machined into a bone surface to provide an accommodating "seating component" for the enlarged rim portion of an implant as disclosed herein, it may be preferable in some cases to eliminate additional anchoring components, and rely on a combination of other anchoring means, such as: (1) bone cement; (2) one or more suture strands that are firmly secured to a flexible anchoring cable that is embedded within the polymeric rim of an implant; and/or, (3) "seating" of the enlarged rim component, within an accommodating groove, trench, or similar structure that has been machined into the supporting bone surface. The level of security and stability that can be provided by this approach can be enhanced by various methods or devices, such as by: (i) creating a bone groove that is angled slightly toward the centerpoint of the implant, to create a "snap"-type fitting of the implant rim into the bone groove; (ii) using a swellable material, a roughened outer surface, and/or similar means to create an implant rim that will "grip" the bone groove more securely; (iii) using mechanical tightening or cinching means, shape-memory components that will shrink slightly when they warm up, or similar means to tighten the grip of the rim on the interior wall or surface of the groove or trench in the supporting bone surface; and, (iv) using other attachment means, including bone cement which can bond to various types of polymers and to other porous materials (such as wire meshes) that can be exposed on the anchoring surface of an implant.

In another preferred example, a stabilizing ring can be provided with one or more segmented, protruding, or other components or surfaces (which can include eyelet devices, mesh materials, etc.) that will extend outside of the flexible polymer component of an implant. This can allow sutures, staples, bone cement, or other means to be used to secure the implant to the supporting bone.

In another preferred example, a first anchoring component that does not contain a flexible polymer component can be securely affixed to a prepared bone surface, by means such as screws, staples, sutures, etc. To provide the surgeon with optimal working space, the initial anchoring steps can be performed and completed before the flexible polymer implant device is inserted into the joint that is being repaired. After that initial anchoring procedure has been completed, the flexible polymer implant can then be inserted into the joint, and either: (i) affixed directly to the first anchoring component; or, (ii) secured in place, in a manner that utilizes the anchoring component to provide greater strength and stability to the entire assembly.

This approach is illustrated in FIG. 6, which shows a type of implant 100A, generally as described above, and having an enlarged outer rim 112A. A stabilizing ring 140A, made of a shape-memory material, is embedded within the polymer material of outer rim 112A. Stabilizing ring 140A has an arc-shaped cross-section which is comparable to a retractable metallic tape measure, as described below.

After the hyaline cartilage has been removed from a bone surface 90, and after a groove 92 (shown in a cross-sectional view in FIG. 6, and sized and shaped to accommodate the rim 112A of implant 100) has been machined into the surface of the bone 90, a surgical implant that can be referred to as an "anchoring subassembly" 160 is securely affixed to the surface of bone 90, along the inner edge of the machined trench 92. Anchoring subassembly 160 comprises "trench-supplementing component" 162, which will effectively help "lock in" the enlarged rim component 112 of a flexible cartilage-replacing implant. If desired, the trench-supplementing component 162 can be provided with an internal stabilizing ring 164, made of a shape-memory, super-elastic, or similar material, embedded within the ring-shaped trench-supplementing component 162. The anchoring subassembly 160 can be securely affixed to bone 90 with the aid of an anchoring disc 166, which will be secured to the bone by a plurality of anchoring means 168 (such as screws, pins, staples, etc.). Anchoring disc 166 preferably should be provided with a shape that is analogous to enlarged washer with an open center, but with an overall shape that corresponds to the overall shape of the implant device (such as the elongated shape of an implant that will replace cartilage that covers a femoral runner surface). The open center will allow bone or scar tissue to grow directly into an "ingrowth surface" (as described above) on the anchoring side of the flexible implant device.

The components shown in FIG. 6 are simplified, for purposes of illustration; for example, to minimize any abrasion or damage to the polymer component of an actual implant, anchoring screws or pins 168 would be countersunk into the anchoring disc 166 or into the bone surface, and the anchoring disc 166 can have a beveled, tapered, or rounded internal edge (alternately, it can be countersunk into a groove that has been machined into the supporting bone, so that the upper edge of anchoring disc 166 is flush with the prepared bone surface). In addition, while anchoring subassembly 160 as illustrated in FIG. 6 is positioned in a manner that is partially nestled into the bone trench 92, it alternately could be positioned outside the bone trench.

After the anchoring subassembly 160 has been fully anchored to the bone, a flexible polymeric implant (as shown in FIGS. 1-3) will be emplaced directly over it, and coupled to it (in FIG. 6, the flexible polymer disc that spans the center portion of the implant is not shown, to simplify the illustration of the anchoring mechanism). If desired, a "snap ring" type of securing mechanism can be used, since the tubular polymer rim 112 that surrounds the stabilizer ring 141 will be flexible. Alternately, a partially-hydrated polymer, which will swell to a larger size when fully hydrated, can be used to form the implant rim 112. If desired, bone cement or a bone-regenerating material can be used to help ensure that the implant rim 112 is firmly anchored within the groove 92 that has been machined into the surface of bone 90.

Accordingly, FIG. 6 illustrates just one of various mechanical designs that can utilize a combination of:
(i) an anchoring subassembly, which will be designed to be firmly and permanently anchored directly to a prepared bone surface, while working space is available to do so (i.e., before the flexible polymer implant is inserted into the joint, via an insertion tube); and,
(ii) a flexible polymer component, which will be affixed to the anchoring subassembly in a manner which utilizes the already-affixed anchoring subassembly to provide a convenient and practical attachment mechanism that will provide a strong and stable mounting system for the flexible polymer component.

In considering the techniques and devices that are disclosed herein, it also should be noted that the anchoring means that can be used for such implants can use combinations of: (i) permanent and nonresorbable components, and (ii) resorbable sutures or other anchoring means, which can be designed to be gradually dissolved by bodily fluids while the ingrowth of bone or scar tissue into a porous anchoring surface of the implant provides permanent anchoring.

### STABILIZING RINGS WITH VARIABLE FLEXURE STIFFNESS

In addition to the use of shape-memory materials to make the stabilizing rings disclosed herein, another design approach is disclosed herein, which utilizes controllable cross-sectional shapes to achieve (or at least facilitate) the types of behaviors and performance results that are desired for cartilage-replacing implants as disclosed herein.

This design approach can be better understood by considering the behaviors of two common household items, which are: (1) inexpensive plastic drinking straws; and, (2) metallic tape-measures.

When a standard plastic straw is bent slightly, it will exert some level of resistance, only until it reaches a point where its circular cross-section is forced to collapse. When it reaches that transition point (which can also be regarded as a failure point), it makes a rapid transition to a flattened cross-section. Once that transition occurs, the straw can be bent easily, such as into a "hairpin" shape, where the cross-sectional shape of the straw, at the apex of the curve, will be effectively flat.

A completely round and tubular straw will be damaged by that type of bending, as can be seen by the ridges, wrinkles, or other deformations that will be created where the plastic became bent. By contrast, a conventional metallic tape-measure (of the type that is stored in rolled-up form inside a convenient case) suffers no such damage, since its cross-sectional shape is only a shallow arc, rather than a complete circle.

Using the conventional scales that are used to describe circles, there are 360 degrees in a complete circle; an arc of 180 degrees is a half-circle; and, an arc of 90 degrees is a quarter-circle. Short metallic tape-measures (up to about 12 feet or 4 meters long) usually have arcs of about 30 to 40 degrees, while longer tape measures (up to about 25 feet or 8 meters) have arcs of about 80 degrees, to give them greater stiffness when extended out to longer lengths.

Regardless of specific dimensions, any metal tape measure is designed to remain straight, and to resist bending forces, when in use and extended, thereby allowing it to be conveniently used to measure things while someone holds the case in one hand, and uses the extended measuring tape in a manner similar to a pointing device. However, that type of stiffness is effective only until the bending force reaches a transition point, which will then force the tape to take a flattened cross-sectional shape. If a tape has been extended beyond the distance its " stiffness level" can support, it will suddenly bend somewhere along its length, and the end of the tape will fall downward. Alternately, when a measuring task has been completed and the tape must be retracted back into the case, the tape will lose its arc shape and transform into a flat layer along its entire length, as it is rolled up and retracted. Either type of transition is non-destructive; a metallic tape measure of this type will be made of a relatively elastic alloy that allows the tape measure to be extended (for use) and retracted (for storage) an unlimited number of times.

Accordingly, one preferred design for stabilizer rings as used herein can utilize an arc-shaped cross-section (similar to the cross-sectional arc of a tape measure), around at least a portion of a stabilizer ring. This design approach is illustrated in FIG. 6, in which the stabilizer ring 140A has an arc shape, which in cross-section looks comparable to a parenthesis. As long as the outer ring 140A (and the implant device 100A which contains reinforcing ring 140A) is in its original manufactured shape, when seen from above or below, stabilizer ring 141 will have a high or even very high degree of stiffness. That is the shape and state it will return to, and remain in, once it is nestled and settled into an accommodating groove that has been machined into a supporting bone surface. The stabilizing ring and the bone groove will be designed to accommodate each other, without generating any stresses or deformation on stabilizing ring 140A. In that form, ring 140A can provide substantial stiffness, which is useful in a stabilizing element.

However, during the insertion step, when the entire flexible implant must be deformed in order to push it into the joint via an insertion tube, the stabilizing ring 141 can transform from its arc cross-section, into a flattened cross-section that will allow almost unlimited bending, in a manner comparable to the way a metal tape-measure becomes flat at some location along its length, and thereafter allows virtually unlimited bending with virtually no resistance, once it passes a transformation (i.e., flattening and collapse) point.

### TWISTED OR BRAIDED ANCHORING CABLES

As mentioned above, an anchoring component embedded within the peripheral rim of a molded polymer component of a cartilage-replacing implant can be made of a flexible cable, having a number of strands with relatively small diameters that are twisted or braided together. That type of twisted or braided cable will have an irregular surface, rather than the type of smooth surface found on extruded metal wires, and on "monofilament" polymer lines (as used in fishing). This will enable a polymer that is molded around a twisted or braided cable to "grip" the surface of the cable more tightly and securely, which can help prevent slipping, sliding, or other displacement of the polymer along the length of the cable. This factor will be reflected in a higher "pullout strength", which is a testing factor that indicates the amount of tensile force required to pull an internal component out of a surrounding material.

A twisted or braided anchoring cable as described herein can be made of:
(i) a biocompatible alloy, such as stainless steel wires, or any of various other known alloys that have sufficient flexibility when created in thin-strand form;
(ii) a high-strength polymer, such as strands of "ultra-high molecular weight polyethylene" (UHMWPE), which have greater strength than steel strands having the same dimensions; or,
(ii) a combination of metal strands and polymer strands, selected to provide an optimal level of stiff-yet-deformable behavior for use in a particular type of implant having known and specific dimensions.

The ends of the twisted or braided anchoring cable can be secured to each other by any suitable means, such as "spot welding", a crimping collar (such as collars 149 and 213, shown in FIGS. 2 and 8), etc. The coupling means that is chosen will not be crucial to this invention, since it will be embedded within the molded polymer component.

This type of multi-stranded cable 190 is illustrated in FIG. 7, which depicts an implant device 180 (shown in the shape of a flat disc, to simplify the illustration), having a molded polymer component 182, which has a smooth and wettable articulating surface 184, and an anchoring surface 186 which is provided with a porous mesh or similar layer 188 which will promote tissue ingrowth for stronger long-term anchoring. A flexible multi-strand cable 190, made of a plurality of thin strands that are twisted or braided together, is embedded within the molded polymer component 182, around its outer rim (periphery).

FIG. 7 also depicts an anchoring peg 192, which is affixed to the flexible cable 190. Anchoring peg 192 is provided with an outer surface having a set of ridges 194 in a "sawtooth" configuration. This will allow such pegs to be pressed into accommodating receptacles (which can also be called sleeves, barrels, or similar terms), in a manner which will allow the pegs to "lock" into position, with no risk of inadvertently coming out. Such receptacles can be emplaced in holes that can be drilled into a bone surface, after damaged native cartilage has been removed from the bone, prior to arthroscopic insertion of the implant device into the joint that is being repaired.

If desired, the internal surfaces of the anchoring receptacles can be provided with a set of corresponding ridges. Alternately, if desired, bone cement can be used, to eliminate any need for anchoring receptacles; and, if desired, the ridged-type surface shown on anchoring peg 192, in FIG. 7, can be replaced by a different type of textured surface, so long as the textured surface is designed to provide better gripping and/or adhesion than can be obtained with a smooth and glossy surface.

In a large implant, such as to repair a knee or hip, a plurality of anchoring pegs can be coupled to anchoring cable 190, at suitable locations around or near the outer rim of implant 180. In a small implant, such as a "button" implant for repairing a finger or thumb joint, a single anchoring peg (or anchoring screw, as described above) can be used.

### OPTIMAL FLEXIBILITY FOR ANCHORING CABLES AND IMPLANTS

If a flexible multi-stranded cable (or a ring or comparable device made of nitinol or any other type of specialized "shape-memory" or "super-elastic" material) is used to provide an anchoring component as described herein that is embedded within a moldable polymeric component in a cartilage-replacing implant, it should have a proper balance between stiffness (which can also be referred to by terms such as rigidity, hardness, etc.), and flexibility (also referred to by terms such as pliability).

As a general statement, the goal is to provide the anchoring cable or other device, and the entire implant (including the molded polymer component) as a unit, with optimized traits that provide both:
(i) sufficient flexibility to allow the implant device to be rolled up or otherwise flexed, compressed, or manipulated into a cylindrical, arc-shaped, or other configuration with a reduced width that will allow the implant to pass through an arthroscopic insertion tube, during implantation; and,
(ii) sufficient stiffness to enable the implant to settle and "seat" onto a prepared bone surface (which can include a trench or groove, machined into the bone surface, to accommodate an enlarged outer rim on the implant) in a manner that will enable the implant to remain stationary and secure for decades, regardless of any loadings or stresses that may be imposed on the joint, including peak loads or stresses that may occur during a fall or other accident.

When considering the balance between those opposing goals, it should be recognized that there is no fixed size limit for arthroscopic insertion tubes. Nevertheless, the diameter of any such tube must be kept as small as possible, to minimize the damage that must be inflicted on tendons, ligaments, muscles, blood vessels, and other soft tissues in the region being repaired. Accordingly, if a reinforced polymeric implant has sufficient flexibility to allow it to be inserted into a joint via the "smallest practical" insertion tube, that flexibility can minimize: (i) the damage that must be inflicted on surrounding tissues during surgery; (ii) the pain and recovery time that must be endured by the patient; (iii) the risk of infection, which will remain a threat until any incisions have fully closed and healed; and, (iv) the risk of creating am undesired post-repair condition, involving unwanted scar tissue, improper tissue regeneration, infection, or similar factors.

The proper balance between flexibility and stiffness, in an anchoring cable, will depend on the size, shape, and insertion site of an implant. As a simple illustration, an implant designed to replace a femoral runner, in a knee, will have very different traits compared to an implant designed to repair a finger joint. To provide anchoring cables that can be positioned at any location along a wide spectrum, with "extremely flexible" at one end and "extremely stiff" at the other end of the spectrum, three physical parameters can be modified and controlled, for any starting material, such as a titanium alloy or a suitable polymer. Those three physical traits are:
1. the thickness of each strand, which can range anywhere from (i) thin and fine wires (such as with diameters less than 0.1 mm) that can be readily bent, to (ii) thick and heavy wires (with diameters greater than 1 mm) that, when aggregated into a cable, can be bent only with the use of tools;
2. the number of strands that will be incorporated into a cable, which in most cases will range between 3 (for relatively thick strands) and about 20 (for relatively thin strands); and,
3. the looseness or tightness of the twisting or braiding structure, in a cable. If a cable with a twisted helical structure made of wire strands is wrapped tightly (such as with several helical turns per centimeter of cable length), it will be stiffer than a cable that is wrapped loosely, within only a single turn (or fraction of a turn) per centimeter of length.

By controlling those dimensional traits, a cable that is manufactured from a particular type of suitable alloy or polymer can be given any desired level of stiffness. Furthermore, a cable can be manufactured from an assortment of strands having different diameters, and/or made of different materials. For example, a cable made with one, two, or three strands of a relatively stiff metal alloy (such as stainless steel) having one or more chosen diameters, combined with a number of very thin strands of a polymer having high tensile strength but low stiffness (such as conventional nylon fibers), can be created with any desired level of stiffness.

Accordingly, a manufacturer can use these controllable options and parameters to create an anchoring cable (either in a continuous loop, or in segments) that balances flexibility against stiffness, at a level that is optimized for any type of implant having a known size, shape, and intended site of implantation.

Since the term "flexible" can be a relative term which can mean different things in different contexts, a "benchmark" standard is provided herein, to determine whether a cartilage-replacing implant qualifies as being "flexible". As used herein, an implant device is deemed to be "flexible" (or, stated in equivalent terms, as having "substantial flexibility"), if the device, as manufactured and assembled in a form that will be removed from a sealed sterile envelope by a surgeon immediately before implantation, meets either or both of the two following criteria:
(1) if it can be flexed (or curled, rolled, bent, etc.), using normal finger strength and without requiring tools, into a configuration that has an "angle of displacement" of at least about 70 degrees. This means that if one edge of the implant is held horizontal on the surface of a table, a surgeon can lift and curl the opposing edge of the implant to an angle of at least 70 degrees from horizontal (i.e., 20 degrees short of vertical), using normal hand and finger strength, without requiring tools; or,
(2) if it can be flexed into a stable and non-damaging configuration where its thickness (which refers to its largest dimension, when looked at in a direction parallel to its longest dimension) is reduced to 80% or less of its thickness in a non-flexed, relaxed state. By way of illustration, if a femoral runner implant (which generally will have a shape and curvature as shown in FIG. 9) or a meniscal implant (which generally will have an "arcuate" wedge shape with a triangular cross-section, as shown in FIG. 25) can be temporarily "straightened out", from a curved or semi-circular shape that was created by a molding or similar manufacturing process, into a more linear flexed shape that can be pushed into a joint through a cylindrical insertion tube, then that implant is regarded as "flexible" if its thickness, when flexed, is only 80% or less of its thickness when relaxed.

This type of flexure can be done with the aid of tools, if desired, since an implant of this type can be shipped and stored in that type of "straightened" configuration, already emplaced within an insertion tube. Accordingly, the important question is not whether tools or a machine were required to flex the implant into that type of straightened configuration, for shipping or storage, but whether the implant, when pushed out of the insertion tube, will quickly revert to its normal curved shape in a manner which was not damaged (or "biased", or otherwise adversely affected) by having to spend time in a flexed and straightened shape, in the insertion tube.

In accord with that principle, steps can be taken, if desired, to minimize the amount of time that a flexible implant will need to spend in a flexed and straightened configuration, inside an insertion tube. For example, rather than storing a flexible implant inside an insertion tube for a "shelf life" that might be measured in weeks or months, steps can be taken to "package and seal" an implant within an insertion tube only a day or two before it is scheduled to be implanted by a surgeon.

Similarly, if desired, steps can be taken to create insertion tubes having desirably controlled curvatures. This can minimize the amount of flexing and stresses that are imposed on a flexible implant, during its time in an insertion tube.

It should also be noted that if a meniscal implant (which includes labral implants, as described above), or any other type of cartilage-replacing implant, is designed to be affixed to tendons, ligaments, or other non-bone tissue, any flexible anchoring cable or similar component which is embedded within its polymeric component generally should not be provided with high levels of stiffness. Instead, the embedded anchoring component should be able to emulate and accommodate the flexibility of the surrounding soft tissue. In this type of arrangement, an anchoring cable made of thin and flexible fibers of a biocompatible polymer with high tensile strength (such as nylon, as one example) is likely to be preferable to a cable made of a metal alloy. In such a case, the function of the cable effectively will be to distribute and allocate any "point-loaded" stresses around a larger area of the implant, thereby converting any localized "peak loadings" that might cause unacceptably high stresses, into lower and more distributed stress levels that will not damage an implant device, even over a span of decades of use.

### RATCHETING KNOTLESS SUTURE ANCHORS

As introduced in the Background section, the Applicant's efforts to develop improved anchoring systems, specifically focusing upon flexible cartilage-replacing implants, led to the realization that a plurality of different but complementary anchoring means, used simultaneously, can provide implants designed for load-bearing joints, such as knees and hips, with better and stronger overall anchoring, compared to the use of only some such anchoring means. Accordingly, in addition to the types of anchoring screws and pegs described above and illustrated in FIGS. 1-5 and 7, this application also discloses the use of a plurality of anchoring sutures, affixed at spaced locations to a reinforcing ring, flexible cable, or similar anchoring component that is embedded around the periphery of an implant. Such a system is illustrated schematically in FIGS. 8 and 9, wherein a plurality of suture strands 210-216 are wrapped around a flexible anchoring cable 212. Anchoring cable 212 is embedded within polymer component 230, in a femoral runner implant 200 as shown in FIG. 9, in a manner which allows the free ends of the suture strands to emerge from the implant 200.

As also introduced in the Background section, that type of suture anchoring can be optimized, for a surgeon's use, by passing the free ends of the suture strands through devices referred to herein as "ratcheting knotless suture anchors". The term "ratchet" (also spelled "rachet", as mentioned above) indicates that:
(i) a suture strand can be pulled through one of these devices in a first "allowed" direction without serious difficulty; and, (ii) a mechanism within the device prevents (or strongly impedes, as described below) the suture strand from being pulled back through the device in the opposite (i.e., blocked, prohibited, or similar terms) direction.

In particular, if each of the anchoring suture strands passes through a ratcheting knotless suture anchor, then the system, as an integrated assembly, will allow a surgeon to take each and all of the anchoring sutures through a process which was described in the Background section by the phrase, "start them all, then snug them all, then tighten them all". That descriptive phrase can be shortened to, "start, snug, tighten", so long as the reader or listener understands that it refers to starting *all* of the strands, before *any* of them are pulled snug; then, tensioning *all* of the strands to a "snug" level, before *any* of them are pulled to a point of being "tight"; then, tightening all of the anchoring sutures, until each and all of them reach a final level of desired tightness. In this manner, the presence of ratcheting suture anchors, positioned at a number of spaced locations around the outer rim of an implant, will help a surgeon position, adjust, "seat", and "smooth out" a cartilage-replacing implant while it is being installed, to help ensure an optimal installation.

Accordingly, attention must turn to various types of ratchet-type mechanisms that offer good candidates for devices that can be miniaturized sufficiently to:
(i) render them well-suited for surgical emplacement and tightening, using arthroscopic and/or laparoscopic tools;
(ii) remain in place for the remaining life of a patient, without creating protrusions or exposed edges which are likely to generate risks of tissue damage, after emplacement.

As described in the Background section, FIGS. 10 and 11 were used to illustrate and describe two different types of "classic" ratchet systems that are well-known, and that are widely used in relatively large devices, such as:
(1) clocks and other mechanical gearing systems, which often use rotating gears that are constrained by spring-mounted "pawl" components, as shown in FIG. 10; and,
(ii) "cam cleat" systems, as shown in FIG. 11, which are used to temporarily secure ropes that are used to adjust the sails, on sailboats.

Passing beyond that known prior art, FIGURES 12-22 illustrate a number of candidate mechanisms which can be miniaturized to a point that will render them suitable candidate mechanisms that can be used to provide ratchet-type control over suture strands that are being used to help secure and anchor cartilage-replacing implants.

Because of its relative complexity and large number of moving parts, the ratchet mechanism shown in FIG. 12 is not regarded as a preferred candidate for use as described herein. Nevertheless, an analysis of that mechanism can provide a "baseline" level of understanding, which can help readers understand how the outer shell functions, and why certain other internal mechanisms are preferable.

FIG. 12 depicts a ratcheting suture anchor 300, enclosed within an outer shell 310. Since this type of anchor is designed to be affixed to a hard bone (rather than being sutured or stapled to soft tissue), the shell component or subassembly 310 will contain a pointed or tapered insertion end 312, and an outer cylinder 314 (which can also be called a sleeve, barrel, hood, or similar terms). The outer shell can have a cylindrical, square, rectangular, elliptical, or other suitable cross-sectional shape.

. As with all suture anchors described herein that are designed to be inserted into hard bone material, either or both of the pointed end 312 and the outer cylinder 314 can be provided with external threads, a ridged sawtooth-like surface, or other any other surface component(s), texture, or shape that will help ensure a stable attachment to hard bone.

One such anchoring means, shown in FIG. 12, comprises a set of barbs 316, which will be made of a spring-type material, which can be generally but not completely flattened against the outer cylinder 314 while the anchor is being inserted into a bone. Once the anchor 300 has reached its proper depth within bone tissue, the slightly curled or angled tips of the barbs 316 will tend to dig into the bone or other tissue, and will generate resistance if a retracting force (such as tensile force imposed on the suture strand) attempts to pull the anchor out of the bone. At least two and preferably three or four barbs 316 should be provided, in a symmetric and outwardly-pointing radial arrangement around the circumference of outer cylinder 314.

If a suture anchor designed for bone emplacement has external threads that are designed to allow the anchor to be screwed into a pilot hole which has been drilled into a bone, then the anchor will also require:
(1) a recessed socket or comparable accommodation, on the upper surface of the anchor shell (i.e., at the end which is opposite the pointed insertion tip), to engage the tip of a hex wrench or comparable driving tool; and,
(2) suitable means for threading one or more suture strands through the anchor device 870, after the anchor device has been emplaced in bone (otherwise, the act of screwing the anchor into the bone would presumably cause the suture strand to become wrapped, several times, around the barrel of the anchor, unless alternate steps are taken). This type of post-emplacement "threading" can be accomplished by emplacing, during manufacture of the device, a small segment of a very thin flexible wire with a loop at one end and a gripping component at the other end, within the ratcheting mechanism of the anchor device, occupying the same position that later will be occupied by a suture strand. After the anchor device has been screwed into a bone surface or otherwise emplaced in soft tissue by a surgeon, the surgeon can insert the free end of suture strand through the small wire loop that emerges from one side of the ratcheting anchor device. The opposite end of the wire segment is then gently pulled, by the surgeon, in a manner that will pull and tow the end of the suture strand through the anchor, so that the suture strand will replace the wire segment, which will be discarded after use to pull a suture strand into position within the ratcheting anchor.

The ratchet mechanism 320 inside anchor 300 is a double-gear mechanism, with a first rotatable gear 330 which interacts with pawl component 332, and a second rotatable gear 340. A compressible spring 334 is shown, which presses pawl 332 against the teeth of gear 330; that type of spring is solely for illustration, and a simpler type of spring mechanism (such as a single-leaf spring, a rubberized elastomeric axle, etc.) normally will be used, to reduce any risk of obstruction by bone chips or other fragments or debris. If desired, pawl components which will block rotation in a non-allowed direction can be provided for both gears.

The teeth of both of the two gears 330 and 340 will attempt to press against each other, due to either or both of two factors: (i) spacing of their axles; or, (ii) by using a spring-loaded mechanism to press gear 340 closer to gear 330. This will cause both sets of teeth to press against (and "bite" into) the surface of braided suture strand 350, which passes between the two gears 330 and 340. In a suitably-designed anchor which interacts with a strong but deformable braided cable (which presumably will be woven from strands of either a ductile alloy, or a high-strength polymer such as a ultra-high-molecular-weight polyethylene or polyaramid fibers), this will ensure that the two gears will grip and hold the suture strand in a manner which will be strong and secure, but which will not cut into, cut through, or damage the suture strand.

An optional pivot or pulley component 360 is also shown in FIG. 12. If desired, it can be used to position and align the suture strand 350. However, that component is not essential, and suture strand 350 can simply be wrapped around gear 330.

This type of anchor 300 can be manufactured as a single unitary device, if it is designed to be driven (such as by gently tapping the anchor into a pre-drilled hole in a bone) into a bone or other tissue in a manner that does not require a rotational screwing motion.

Alternately, a ratcheting anchor can be designed and manufactured to include: (i) an outer component which will be emplaced first, and which can be called a receptacle or similar terms; and, (ii) an insert component, which will be inserted into and coupled to the receptacle. If desired, the receptacle component can be provided with external screw threads on a portion of its outer surface. Internally, it can contain a sawtooth surface, bayonet-type fitting, or other suitable component or means, to allow the insert component to be pushed into the receptacle or sleeve in a manner that will lock the insert in place.

FIG. 13 depicts an alternate design for a ratcheting suture anchor 380. This design uses a similar pointed or tapered outer shell 382 with anchoring barbs 384. Internally, it contains two axle-mounted non-circular (cam shaped) gripping gears 386 and 388, which will contact and press against suture strand 390, which passes between the two gears 386 and 388. Both of the gears will be mounted on their axles using a suitable spring-loading mechanism, such as a coiled spring or an elastomeric polymer component, to rotate at least one and preferably both of the two gears toward each other, so that their toothed or otherwise irregular surfaces will always be subjected to at least some level of force which pushes the two gears inwardly, toward each other. Accordingly, this mechanism will allow a braided suture strand to be pulled through the gears in one direction with little resistance; however, if the suture strand attempts to travel in the other direction, the teeth and gears will grip the surface of the suture strand, in a manner directly comparable to the types of "cam cleat" devices that are used to secure ropes on sailboats, as described in the Background section.

It should also be noted that either or both of the two gears can be mounted in different ways, in a miniaturized device such as a suture anchor. For example, rather than being mounted on spring-mounted axles, FIG. 14 depicts a cam cleat system 400 in which both of the two cam-shaped gears 420 and 430 are mounted at the ends of spring-type extensions 412 and 422, which can be mounted on small axles if desired, or which can be simply molded from a stiff but non-rigid polymer, affixed to the interior surfaces of the anchor shell 410. Alternately or additionally, the two gears 420 and 430 can be positioned within a sleeve component 440 which is made from an elastomeric polymer having substantial thickness. Because of the sizes, dimensions, and directly-facing placement of the gears 420 and 430 between the walls of the elastomeric sleeve 440, the sleeve will press the ridged faces of the two cam gears 420 and 430 against each other, causing them to squeeze the suture strand 450 between the two gear faces. The suture strand 440 can be pulled through the gears 420 and 430 in the direction indicated by the block arrow in FIG. 14, with minimal or only moderate resistance. However, if a retracting force tries to pull the suture strand in the opposite and non-allowed direction, any movement by the suture strand will also pull the two cammed gears downward, and closer together, as they rotate around the attachment points at the bases 422 and 432 of the two gear components 420 and 430. This will cause the toothed gear faces to press hard against each other, in a manner that will effectively lock the suture strand in position, and prevent it from traveling in the non-allowed direction.

FIG. 15 depicts a ratcheting suture anchor 470 containing a single cam-shaped gear 472, mounted at its base 474. Rather than interacting with a second gear, the toothed surface of gear 472 will interact with a sawtoothed or other non-smooth surface 476, which has been molded into an interior wall of the suture anchor shell. In this manner, a single movable gear 472 can establish the same type of ratcheting control, over a braided suture strand 479, that is provided by the two-gear systems shown in FIGS. 13 or 14.

FIG. 16 depicts a ratcheting suture anchor 490 which comprises a cylindrical sleeve 492 that contains numerous stiff bristles 494, lining the internal surface of the sleeve 492. All of the bristles 494 are angled (the term "biased" can also be used) toward the outlet end 496 of the sleeve 492. A braided suture strand (not shown in FIG. 16) can be pulled through sleeve 492, toward outlet end 496, with low or moderate resistance. However, if a tensile force attempts to pull the braided suture strand in the opposite direction, at least some of the bristles will become lodged in the interstitial spaces in the surface of the braided strand, and will prevent the strand from moving in the non-allowed direction.

Another type of knotless anchor, in which a crimping mechanism and action controlled by a surgeon creates a ratcheting effect, is depicted in FIG. 17 (which shows uncrimped device 500) and FIG. 18 (which shows the same device 500, after it has been partially embedded in a bone surface 499 and has been partially crimped).

In its uncrimped, undeformed shape, anchor 500 comprises: (1) a generally cylindrical barrel portion 510, which has a suture conduit 512 (which can also be called a tunnel, orifice, passageway, or similar terms) passing through it longitudinally; and, (ii) a pointed or wedge-shaped segment 530, which has a generally conical or tapered shape that will allow the tip to be driven into a bone, and which also has a conduit 532 passing through it. The two main segments 510 and 530 are coupled to each other by a relatively thin segment or "hinge" of deformable material at juncture 520 (depicted as a circle, in FIG. 17, for purposes of illustration, since it will provide a "pivot point" comparable to a hinge).

A single anchoring suture strand 550 will be laced through both of the conduit segments 512 and 532, which will have their adjacent openings aligned, in both the uncrimped and crimped arrangements. One end of suture strand 550 will be securely affixed to a surgical implant device (such as implant device 200, shown in FIGS. 8 and 9 but not in FIGS. 17 or 18), while strand end 552 will be a "free end" which can be gripped by a surgeon's tool.

Anchoring device 500 also is provided with a "crimping ramp" 540, which has a notched, ridged, sawtooth, or other engaging surface 542, and a crimping corner or edge 544. As indicated in FIG. 18, when anchoring device 500 is bent and crimped, upon installation, the edge or corner 544 of crimping ramp 540 will press and pinch against one side of the suture strand 550; if desired, an accommodating slot can be provided as part of the "mouth" end of conduit 532 which passes through pointed segment 530.

Accordingly, when anchoring device 500 is first emplaced into bone surface 799 (this can be done with the aid of a pre-drilled pilot hole when appropriate, or the anchor can be driven into a bone surface with no pilot hole, by using a device comparable to a stapler or nail gun), there will be relatively little or no crimping together of the two segments 510 and 530, and the surgeon will be able to pull on the free end 552 of anchoring suture 550, with relatively little resistance.

As the surgeon begins to initially tighten suture strand 550 into a gentle and non-final "snugged" level of tension and tightness, the tension exerted on the strand 550 (leading to the immobilized implant device, in a nearby location on the surface of the bone) will begin to initiate a bending and crimping process, which will cause the barrel component 510 to bend or fold over, moving it closer to pointed segment 530. As the extent of bending and crimping between the anchor segments 510 and 530 increases, the edge or corner 544 of crimping ramp 540 will begin to press against and pinch the suture strand 550, in a progressively tighter manner. When the surgeon has completed the final tightening adjustments for that anchoring suture strand, he or she can press or tap down the exposed upper corner of the anchor 500, in a way which will increase the pressure imposed by the edge or corner 544 of crimping ramp 540, against suture strand 550, thereby effectively locking suture strand 550, inside the conduit passing through anchoring device 500, with a desired level of final tensioning.

Various other modifications can be made to this type of crimping anchor device, if desired. For example, a second suture conduit can be provided, either within pointed component 530, or as a groove which travels along an external surface, to enable easier pulling and travel of the free end 552 of suture strand 550. However, at the current time, prior to actual fabrication and testing of such devices, the Applicant herein anticipates that: (i) such a conduit will not be necessary; (ii) an experienced orthopedic surgeon will not have serious difficulty in pulling a suture strand through a relatively tight gap between a bone surface, and an anchoring device; and, (iii) the absence of any such additional conduit can help ensure that the tension in the suture strand, created by the surgeon during installation of the implant, will last until tissue ingrowth into the anchoring surface of the implant has reached a mature and stable level.

Another type of knotless ratcheting suture anchor 600 is depicted in FIGS. 19 and 20. To more clearly depict the various non-planar surfaces that will interact with each other, both the cutaway elevation (side) view shown in FIG. 19, and the plan (top) view shown in FIG. 20, should be regarded as semi-schematic or semi-exploded views, in which the separation shown between the components is exaggerated.

Anchor 600 comprises an outer shell 602, with insertion tip 604, barbs 606, and upper end 608. The main components of the ratchet mechanism include: (i) a seating component 610 (also simply called seat 610, for convenience), which will be affixed to a first side of anchor shell 602; (ii) a retainer device 620, which also can be called a washer, grommet, or similar terms, and which will be affixed to an opposed or angled second side of shell 602; and, (iii) a movable component, referred to herein as a "brake" 630 (it can also be thought of as a bead, ball, or similar terms). The movable brake component 630 is trapped between the seat 610 and the retainer 620. It can be pulled and moved in either direction, but only until it presses against either the seat 610, or the retainer 620. The movable brake 630 has a suture strand 695 affixed to it as described below; strand 695 has an implant end 697 (which is coupled to an implant device( and a free end 699 (which can be gripped and pulled by a surgeon's tool).

As shown more clearly in FIG. 20, this ratchet mechanism provides:
(i) an entry orifice 612, which passes through seat 610 and which allows suture strand 695 to enter one side of anchor device 600; and,
(ii) an exit orifice 622, which passes through retainer component 620, and which allows the free end 699 of suture strand 695 to exit the anchoring device 600.

Within the ratchet subassembly, suture strand 695 will be wrapped in a loop around movable brake component 630, in the manner illustrated in FIGS. 19 and 20. This configuration, and the forces exerted on the movable brake 630 by suture strand 695 (which generally will be much more taut and tight than the loose and relaxed configuration shown in FIGS. 19 and 20) will trap the movable brake 630 in a pathway that allows it to move somewhat, but only in a limited and constrained manner, back and forth between the seat 610 and the retainer 620.

A seating surface 614, which is part of seating component 610, is sized and shaped in a manner that will conform to the size and shape of the movable brake 630. Accordingly, brake 630 will settle into, and press firmly against, seating surface 614, when tension is applied to the implant end 697 of suture strand 695. That seating action, which will occur when a tensile force tries to pull suture strand 695 toward the implant device, will effectively pinch, grip, and hold the suture strand 695 between brake 630 and seating surface 614, in a manner which will prevent suture strand 695 from being pulled toward the implant device to which the strand 695 is attached.

To help ensure an optimal seating interface between movable brake 630 and seating surface 614, conforming and accommodating surfaces should be provided on: (i) the seating surface 614; and, (ii) the side or surface of the movable brake 630 that will directly contact and press against seating surface 614. If desired, rounded surfaces can be used, with a generally spherical brake or bead, and a generally hemispherical or "rounded saucer" seat. However, round shapes tend to pose a greater risk that a brake or bead of this type might become stuck (or jammed, wedged, lodged, or similar terms) in the seating surface, compared to accommodating conical shapes. Accordingly, unless and until testing indicates otherwise, conical shapes for the seating surface and the brake component are deemed to be somewhat preferable over spherical or hemispherical shapes. In addition, it also should be noted that the suture strand, passing through the entry and exit orifices and through the bead as well, will help ensure that the brake component will remain properly aligned with the seating component.

The brake or bead component 630 surrounds and encloses a pathway 632 (which can be called a hole, tunnel, orifice, etc.) which will allow suture strand 695 to be:
(1) inserted all the way through the brake component 630, in a left-to-right direction when illustrated as in FIGS. 19 and 20;
(2) looped halfway around the brake, as illustrated in FIGS. 19 and 20; and,
(3) inserted through the brake pathway 632 a second time, in the same direction as the first insertion.

This will create a continuous loop of suture strand material, which will encircle half of the brake or bead 630. In this manner, the suture strand is provided with an ability to directly pull and move the brake in either direction, i.e., either: (1) toward either the seating component 610, shown on the left sides of FIGS. 19 and 20; or, (2) toward the retainer 620, shown on the right sides of FIGS. 19 and 20. However, as soon as brake 630 "bumps into" and presses against either the seat 610 (if being pulled back toward the implant device), or the retainer 620 (if being pulled away from the implant, by a surgeon using a tool), no further travel of the brake is possible; accordingly, the suture strand can continue to travel, only if the entire segment of strand material (including the loop) is able to slide along any and all surfaces it contacts, including: (1) the entry orifice 612 provided by seat component 610; (2) the internal surfaces of brake pathway 632; (3) the outer surface of brake 630; (4) the internal surface, once again, of brake pathway 632; and; (5) the internal surface of exit orifice 622, which passes through retainer 620.

In direct contrast to the "snug and conforming" seating interface between brake 630 and seating component 610, one or more small bump- or nodule-type protrusions 624 (which can be located either on the "retainer side" of brake 630, or on the brake-contacting interior surface of retainer 620) will have the opposite effect, and will prevent brake 630 from pressing against retainer 620 in a snug and/or conforming manner. Therefore, even when brake 630 is being pulled directly into and against retainer 620, by a tensile force exerted on the free end 699 of suture strand 695, the suture strand 695 will NOT become caught, pinched, or otherwise gripped and held by the very small contact points that will be created between brake 630, and the interior surface of retainer 620, if small bumbs, nodules, or other protrusions are provided, either on the "retainer side" of the brake component, or on the retainer surface, to prevent the brake from nestling into and resting securely against the retainer.

As a result, the surgeon will be able to pull the suture strand through the anchoring device 600 in one direction, as indicated in FIG. 20, but tension from the implant will not be able to pull the suture strand back through the anchoring device in the opposite direction, toward the implant device. Accordingly, this will generate and provide the type of ratcheting mechanism and effect that is desired, in this type of suture anchor.

It also should be noted that a movable brake component that has had a suture strand threaded through it, in the looping manner shown in FIGS. 19 and 20, can be pressed into an anchor of this type, after the anchor has already been driven into a bone or affixed to soft tissue, if the strand entry and strand exit orifices are positioned at the bottoms of crimped slits that extend upward to the top surface of the anchor. This could make it convenient for a surgeon to:
(1) emplace an anchor, such as by screwing an anchor having external threads into a hard bone;
(2) use a tool to press the suture strand into a crimped slit in the anchor shell which leads to the strand entry orifice; (3) use the tool to pull the suture strand in the direction of the implant, until the brake component settles into the seating surface inside the anchor device;
(4) press the other end of the suture strand down into the exit orifice, through a crimped slit, while the brake component remains inside the anchor device.

Two additional and somewhat similar rachet mechanism designs are illustrated in FIG. 21 (which includes panels 21 A and 21B), and in FIG. 22 (which includes panels 21A and 21 B). In both drawings, the "A" panel illustrates travel of the suture strand in the allowed direction, and the "B" panel shows that the suture strand has become pinched and gripped, in a manner which prevents travel of the strand in the non-allowed direction.

FIG. 21 depicts a rachet anchor 700, having:
(i) a seating component 710, which provides a strand-entry orifice 712 and a seating surface 714;
(ii) a retainer component 720, which provides a strand-exit orifice 722, and which has a brake-contacting surface or structure which will not grip and bind suture strand 749; and,
(iii) a movable brake component 730, which can travel only a limited distance, in a constrained pathway, between the seating component 710 and the retainer 720.

In this embodiment, movable brake component 730 does not have a suture pathway passing through the center of the brake component. Instead, it has a coupling component 732, affixed to the side of the brake 730. That coupling component can have the suture strand wrapped around it, in a loop, as described above; alternately, it can be designed for use with a thick and radially-compressible suture strand having a relatively large diameter that will cause the suture strand to press against the interior surface of the passageway through coupling component 732. As a third alternative, coupling component can have a narrow groove or gap passing through an outer wall, to allow the brake component to be squeezed and pressed onto a suture strand from the side, to eliminate the need to thread the suture strand through a small fully-surrounded orifice.

The rachet anchor 750, shown in FIG. 22, has a similar seat assembly 760, retainer assembly 770, and movable brake 780. However, in this embodiment, suture strand 790 is gripped and held by the interaction, not between two smooth surfaces which are being pressed against each other, but by prongs, or by ridged, textured, or other irregular surfaces which are being pressed against each other.

FIG. 23 depicts a ratcheting knotless suture anchor 800, which is assembled *in situ* (i.e., inside a joint, limb, or body), by coupling together: (i) an anchoring subassembly 810; and, (ii) a ratchet subassembly 820. The anchoring subassembly 810 shown in FIG. 23 comprises a flexible "skirt" 812 (which can also be called an apron, sheet, or similar terms), which can be readily punctured and penetrated by devices such as staple points, suture needles, etc. Accordingly, this type of skirt can be secured to either: (i) soft tissue, by means such a staples or sutures; or, (ii) hard bone, by means such as screws, pins, etc. Preferably, the polymer used to make the skirt should be mode of or reinforced by, a woven, braided, or similar mesh or comparable material, to prevent rips and tears from "propagating" outwardly from any puncture sites.

The skirt component 812 has a coupling device or subassembly 314 securely affixed to the skirt. The coupling device has a vacancy or receptacle which is designed and sized to receive and accommodate ratchet subassembly 820, in a secure manner that allows the ratchet subassembly 820 to be firmly and permanently affixed to the anchoring subassembly 810, after the anchoring subassembly 810 has been fully emplaced and completely anchored within a joint, limb, or body. This will allow a complete set of anchoring subassemblies 810 to be inserted into a surgical site, properly positioned, and fully anchored to either hard bone or soft tissue, while the operating field remains relatively uncluttered (i.e., before the implant device itself, or any of the ratcheting subassemblies 820 or their suture strands, have been inserted into the joint, limb, or other site that is being operated upon). After each ratcheting subassembly 820 is positioned over or adjacent to its anchoring subassembly 810, the two subassemblies can be coupled together, by using any suitable type of coupling interface (such as accommodating threaded or slotted surfaces, a "bayonet" fitting, a "snap cap" which uses a metal "split ring" or molded components made of non-rigid plastic, etc.).

FIG. 23 does not illustrate any particular type of ratcheting mechanism, inside ratchet subassembly 820, since any of the types of mechanisms shown in FIGS. 12-22 (or, indeed, any of various other known types of ratchet mechanisms) can be selected and used. Instead, FIG. 23 merely illustrates a suture strand 822 which enters ratchet subassembly 820 via an entry orifice 824, which passes through ratchet subassembly 820, and which exits via an exit orifice (not shown) on the opposite side of subassembly 820.

### SURGICAL KITS IN SEALED STERILE WRAPPERS

This disclosure also discloses another type of "article of manufacture", comprising "surgical kits" which contain:
(1) one or more implant devices with a flexible anchoring ring or cable as disclosed herein; and/or,
(2) at least one ratcheting knotless suture anchor, as disclosed herein,
which are held inside a sealed envelope (or other comparable sealed container) that keeps the surgical components sterile until the kit is opened, during a surgical operation.

These types of surgical kits, which use sealed airtight and watertight envelopes to keep any contents sterile until the envelope is opened by a surgeon, in an operating room, immediately prior to use, are used in nearly all types of surgery that involve implantable devices which are designed to remain inside a patient's (or animal's) joint, limb, or body after the surgery is completed. Such implantable devices are distinct from surgical tools, which typically are designed for multiple uses, and which are designed to be sterilized after each use (usually in an autoclave or comparable device).

Accordingly, FIG. 24 depicts a surgical kit 850, which includes a sealed envelope or comparable enclosure 860, typically created by gluing or heat-sealing a transparent front layer 862 (made of clear plastic, which allows the contents to be visually inspected at any time) to a backing layer 864 (usually opaque, to make it easier to see and read any labeling, instructions, pictures, or other information printed on the back surface). FIG. 24 shows one corner of the clear front layer 862, being peeled up from back layer 864. The two layers are shown in that manner, solely to illustrate those layers; because of the strength and tightness of the glue used to seal the layers together in these types of surgical kits, it usually in impractical or even impossible to peel the layers away from each other, and such envelopes usually need to be cut open.

In the example shown in FIG. 24, envelope 860 encloses and protects a flexible cartilage-replacing implant device 870, which is sized and shaped for use in replacing the hyaline cartilage on a femoral runner, in a knee joint. This example shows six suture strands 872, emerging (at spaced locations) from the outer rim of implant device 870 (for example, each anchoring suture can be wrapped around or tied to a flexible anchoring cable that is embedded within the outer rim of the molded polymeric component, as illustrated in FIGS. 2, 8, and 9). Each suture strand 872 has a ratcheting mechanism 874 coupled to it, and each ratcheting mechanism is designed to be coupled to an anchoring component 880, after the anchoring component has been driven into a bone surface. The ratcheting mechanisms 874, and the anchoring components 880, are illustrated with exaggerated sizes, to simplify the illustration.

Accordingly, the surgical kits disclosed herein can include either or both of the following types of devices:
1. one or more ratcheting knotless suture anchors, which can have either:
   (a) suture strands already emplaced within their racheting mechanisms; or,
   (b) threading aids, which can be, for example, thin and flexible wires are provided with loops for suture strands, at one end, and with gripping means (which also can be loops, if large enough to hold a tip of a pliers-type tool) at the other end;
      and which can be either: (i) unitary systems, ready for installation; or, (ii) component systems, which provide two or more components that are designed to be assembled *in situ,* inside a patient's joint, limb, or body;
      and/or,
2. a flexible implant device (such as, for example, an implant designed to replace damaged hyaline or meniscal cartilage) which:
   (a) has sufficient flexibility to allow it to be rolled or otherwise manipulated into a cylindrical or compacted form that can be inserted into a joint or body via an arthroscopic or laparoscopic insertion tube; and,
   (b) contains a flexible anchoring component (such as a ring of shape-memory or super-elastic material, or a segment of flexible cable) which is embedded with a molded polymer component, and which is designed, positioned, and suited to allow a plurality of bone- and/or tissue-anchoring devices to be securely coupled to said flexible anchoring component.

If desired, these types of kits can also include other components and/or devices, such as, by way of example:
i. anchoring screws or pegs, and anchoring receptacles;
ii. segments or coils of specialized suture material designed for use as anchoring sutures;
iii. segments of mesh or other material that can be used to reinforce (or secure an implant to) damaged tissue that is being repaired; and,
iv. any tools, devices, or supplies that would be useful in an operation involving the contents of such a kit, and which normally would not be readily available in typical clinics or operating rooms where the kit-utilizing surgery would be performed.

### HANDLING LONG THREADS, AND PRESERVING A NATURAL "FEEL"

All of the ratchet mechanisms disclosed herein will allow a suture strand to pass entirely through an anchor device, in a manner which will provide a "free end" of the suture strand. This can provide several significant benefits, compared to the type of rotating ratchet mechanism disclosed in the published applications by Van der Burg et al, cited above.

First, the "direct pass-through" nature of the ratchet mechanisms disclosed herein will allow substantially longer suture strands to be used, for initial anchoring and preliminary reinforcing of a cartilage-replacing implant device, compared to a rotating ratchet system which would need to stuff long suture strands inside miniaturized cylinders that are kept as small as possible. A set of relatively long and directly-accessible suture strands, positioned at key locations around the periphery of an implant, will effectively provide a set of "handles" that can be used by a surgeon to help the surgeon manipulate and position an implant, inside a joint which is being manipulated with only limited arthroscopic access.

It should also be noted, as mentioned above, that the suture strands which are coupled to an implant can be color-coded, to provide a surgeon with an additional set of visual cues, to help the surgeon complete the surgical procedure quickly and effectively. Accordingly, if relatively long segments of surplus strand material are used, it will be easier for a surgeon to see and identify each such strand, typically via a video monitor coming from a miniaturized camera lens inside a surgical field that has blood, saline solution, and debris flowing through it.

Secondly, since all of the ratchet mechanisms disclosed herein will allow the "free end" of a suture strand to be grasped and pulled by a surgeon, these designs can preserve a normal and natural "feel", which most arthroscopic surgeons would prefer to have, during a tightening procedure. By contrast, a rotating ratchet mechanism, as disclosed in published application 2010/0063542 (Van der Burg et al) will need to be driven by some type of wrench or similar powered tool, which cannot generate the same type of tension-sensing "feel" that a grip on the free end of a suture strand can provide

.Furthermore, in a rotating ratchet system which must be driven by a powered wrench, there is some level of risk that the accumulation of a significant length of suture strand, in a narrow and tightly constrained gap between an internal rotating device and a surrounding sleeve, might cause the rotation and responsiveness of the mechanism to be altered, and distorted, in ways that cannot be fully predicted or controlled if a substantial length of suture strand is involved.

Finally, it should be noted that the relatively simple "direct pass-through" nature of the ratchet mechanisms disclosed herein can enable various designs and methods for momentarily releasing the grip of a ratchet mechanism on a suture strand, in a way that will allow the tension in the strand to be reduced, if necessary. As just one example, a small sleeve made of smooth-surfaced plastic, with a slit passing through it lengthwise, can be fitted onto the surface of a suture strand, immediately "above" or "below" a ratchet mechanism. If the smooth sleeve is pushed or pulled into the ratchet mechanism, it can create enough separation, between two gears, crimping curfaces, or similar devices or surfaces, to enable a suture strand to be pulled backward through the ratchet mechanism.

Accordingly, when restated in terms suited for patent claims, the types of anchoring devices disclosed herein, to be covered as part of this invention, must be designed, sized, and suited for permanent attachment to at least one type of internal tissue (which can be either hard bone or soft tissue), wherein the anchoring device has a passageway and a ratcheting mechanism which will enable a suture strand to pass through the anchoring device in a manner which will: (a) enable a surgeon to gradually snug and then tighten a suture strand, by pulling the suture strand through the anchoring device in a first direction; and, (b) prevent the suture strand from traveling through the anchoring device in an opposing direction, which if not prevented would allow the suture strand to become looser; and wherein the anchoring device enables a suture strand to be initially pulled snug to a first level of positioning tension, and later tightened to a second level of final tension during a final tensioning procedure.

### USE FOR OTHER TYPES OF SURGICAL REPAIRS

The types of ratcheting suture anchors disclosed herein cam also be used for other types of surgical uses, other than anchoring implants that will replace cartilage in articulating joints.

As one example, these types of ratcheting suture anchors can be used by surgeons to help them repair torn or otherwise damaged rotator cuffs, in human shoulder joints. As is well-known to orthopedic surgeons, a typical rotator cuff tear involves the detachment of certain tendon and ligament structures, from the humerus (i.e., the long bone in the upper arm). When that type of tear occurs, the torn tendons and ligaments will tend to retract deeper into the shoulder joint, generally toward a patient's shoulder blade. Accordingly, most rotator cuff repairs require a surgeon to gently but firmly pull torn tendon and ligament segments in an "outward" (lateral) direction, so that they can be reattached to the enlarged and rounded bone structure (called "the greater tuberosity of the humerus") at the upper end of the humerus. Once those damaged tendon and ligament segments have been pulled in an outward direction, back toward their proper position, the surgeon does his best to reattach them to the end of the humerus. Accordingly, small and convenient suture anchors which contain ratcheting mechanisms, as disclosed herein, can be very useful to surgeons who perform rotator cuff repairs.

Rotator cuff repairs offer just one example of how knotless suture anchors that have ratcheting capability, which can be exerted by simply pulling the free end of a suture strand through the anchor, would be very useful in various types of surgical and "sports medicine" repair of various types of connective tissues. If these types of ratcheting suture anchors become readily available to surgeons, other uses at other locations will also be identified and tested.

### FLEXIBLE CABLES FOR ANCHORING MENISCAL IMPLANTS

FIG. 25 depicts a meniscal implant 900, which has a shape that can be referred to as an arc-shaped (or "arcuate") wedge, somewhat similar to a section from a tangerine or other citrus fruit. Regardless of whether a native meniscal segment is on the interior or lateral side of a knee joint, it will have three important surfaces, indicated in FIG. 25 as:
(i) an upper articulating surface 902, which will be smooth and "lubricious", and which will press and articulate against the rounded bottom surface of a femoral runner
(ii) an outer peripheral surface 904, generally in the shape of a vertical cylindrical segment, which will not articulate against cartilage, and which instead will be coupled to the tissues which form a "knee capsule" (i.e., the tendons, ligaments, and membranes which enclose and hold in the synovial fluid, which lubricates a knee joint); and,
(iii) a smooth and lubricious lower surface 906, which is roughly planar, and which rests upon and slides against an upper surface of a tibial plateau. This lower surface is bounded and defined by a first arcuate interior edge, a second arcuate peripheral edge, and opposing tips 908 and 909 where said first and second arcuate edges meet.

Accordingly, meniscal implant 900 is anchored, stabilized, and reinforced with the aid of two different elongated flexible reinforcing members, which are depicted by "beaded lines" 922 and 932 in FIG. 25. Reinforcing member 922 is positioned along or near the "lower" outer peripheral edge 920 of the meniscal wedge 900, and reinforcing member 932 is positioned along or near the "upper" outer peripheral edge 930 of the meniscal wedge 900.

For simplicity of illustration, both of the reinforcing members 922 (lower) and 932 (upper) are depicted as extending out of and beyond the two opposing tips 908 and 909 of the flexible polymer material. In actual practice, either or both of the lower and upper reinforcing members 922 and 932 are likely to be coupled, at each end, to a plug-type device that is coupled directly to (or positioned closely adjacent to) the two tips 908 and 909 of the meniscal wedge. These types of plug-type anchoring components can be set into small holes that have been drilled into a tibial plateau, in a manner that provides a larger, more distributed, and therefore stronger anchoring interface than can be achieved by a single screw or pin.

In addition, it likely will not be essential, in all cases, to provide two different reinforcing members along both the lower and upper peripheral edges of a meniscus. For example, if the entire polymer component is reinforced by a fiber mesh, that mesh can eliminate the need for a second elongated reinforcing member along the upper peripheral edge of the meniscal wedge.

Thus, there has been shown and described a new and useful design for surgical implants for replacing and repairing cartilage. Although this invention has been exemplified for purposes of illustration and description by reference to certain specific embodiments, it will be apparent to those skilled in the art that various modifications, alterations, and equivalents of the illustrated examples are possible. Any such changes which derive directly from the teachings herein, and which do not depart from the scope of the invention as defined by the claims, are deemed to be covered by this invention.

## Claims

1. An implant device for repairing damaged cartilage in a mammalian joint,
said implant device being a meniscal implant (900) sized and shaped to replace meniscal cartilage, comprising
a smooth and lubricious lower surface (906) sized and shaped to articulate in contact with a tibial plateau, said lower surface (906) being bounded and defined by an arcuate interior edge, an arcuate peripheral edge (920) and opposing tips (908, 909) where said arcuate interior and arcuate peripheral edges meet,
a smooth and lubricious upper articulating surface (902), sized and shaped to articulate in contact with a femoral runner, after implantation;
an arcuate peripheral surface (904), sized and shaped to contact soft tissue in a knee capsule, after implantation; and
an arcuate upper edge (930) where said upper articulating surface (902) and said arcuate peripheral surface (904) meet;
at least one lower elongated reinforcing member (922) embedded in said arcuate peripheral edge (920); and,
at least one tissue anchoring component coupled to said elongated reinforcing member (922),
**characterized in that**
an upper elongated reinforcing member (922) having a plurality of tissue anchoring components coupled thereto is coupled to or embedded within said arcuate upper edge (930).

2. The implant device of claim 1, wherein said at least one tissue anchoring components is selected from a group consisting of:
a. a protrusion that will extend into a prepared hole in a bone surface;
b. a component that can be secured to a head of a bone screw;
c. one or more strands of wire
d. one or more strands of suture material;
e. a segment of reinforcing mesh that is partially embedded within the molded polymeric component and which extends out of said molded polymeric component in at least one location; and,
f. at least one eyelet device, each provided with an aperture.

3. The implant device according to any one of the preceding claims, wherein at least one said reinforcing member (922, 932) is made of a material selected from the group consisting of:
a. a shape-memory alloy having at least one temperature-dependent physical or behavioral characteristic;
b. a shape-memory polymer having at least one temperature-dependent physical or behavioral characteristic;
c. a super-elastic material;
d. a multi-stranded cable; and,
e. a high-strength polymer.

4. The implant device according to any one of the preceding claims, wherein the implant can be flexed and deformed, without requiring tools and without suffering damage, into an elongated shape having a width of about 75% or less of its relaxed width.

5. The implant device according to any of the preceding claims, wherein said upper elongated reinforcing member (932) that is coupled to or embedded within said arcuate upper edge (930) has at least one end which can be anchored directly to bone or soft tissue.

6. The implant device according to any of the preceding claims, wherein said upper elongated reinforcing member (932) that is coupled to or embedded within said arcuate upper edge (930) has at least one end which is coupled to said elongated lower reinforcing member (922).

## Patentansprüche

1. Implantatvorrichtung zur Reparatur beschädigten Knorpels in einem Säugetiergelenk, wobei die Implantatvorrichtung ein Meniskusimplantat (900) und so dimensioniert und geformt ist, dass sie einen Meniskusknorpel ersetzt, aufweisend
eine glatte und lubrizierte untere Oberfläche (906), die so dimensioniert und geformt ist, dass sie in Kontakt mit einem Tibiaplateau gelenkig interagiert, wobei die untere Oberfläche (906) durch einen bogenförmigen inneren Rand, einen bogenförmigen umlaufenden Rand (920) und gegenüberliegende Spitzen (908, 909) begrenzt und definiert ist, an denen sich der bogenförmige innere und der bogenförmige umlaufende Rand treffen,
eine glatte und lubrizierte obere Gelenkfläche (902), die so dimensioniert und geformt ist, dass sie nach der Implantation in Kontakt mit einer femoralen Gelenkfläche gelenkig interagiert;
eine bogenförmige umlaufende Oberfläche (904), die so dimensioniert und geformt ist, dass sie nach der Implantation mit weichem Gewebe in einer Kniekapsel in Kontakt ist; und
einen bogenförmigen oberen Rand (930), an dem sich die obere Gelenkfläche (902) und die bogenförmige umlaufende Oberfläche (904) treffen;
mindestens ein unteres längliches Verstärkungselement (922), das in dem bogenförmigen umlaufenden Rand (920) eingebettet ist; und
mindestens eine Gewebe-Verankerungskomponente, die mit dem länglichen Verstärkungselement (922) gekoppelt ist,
**dadurch gekennzeichnet, dass**
ein oberes längliches Verstärkungselement (922), das mehrere mit diesem gekoppelte Gewebe-Verankerungskomponenten hat, mit dem bogenförmigen oberen Rand (930) gekoppelt oder in diesen eingebettet ist.

2. Implantatvorrichtung gemäß Anspruch 1, wobei mindestens eine Gewebe-Verankerungskomponente aus der Gruppe ausgewählt ist, die besteht aus:
a) einem Fortsatz, der sich dann in ein vorbereitetes Loch in einer Knochenoberfläche erstreckt;
b) einer Komponente, die an einem Kopf einer Knochenschraube befestigt werden kann;
c) einem oder mehreren Drahtsträngen;
d) einem oder mehreren Strängen Nahtmaterial;
e) einem Segment aus Verstärkungsnetz, das in der geformten Polymerkomponente eingebettet ist und das sich an mindestens einem Ort aus der geformten Polymerkomponente heraus erstreckt;
und
f) mindestens einer Ösenvorrichtung, die jeweils mit einer Öffnung ausgestattet ist.

3. Implantatvorrichtung gemäß einem der vorhergehenden Ansprüche, wobei mindestens ein Verstärkungselement (922, 932) aus einem Werkstoff hergestellt ist, das aus der Gruppe ausgewählt ist, die besteht aus:
a) einer Formgedächtnislegierung, die mindestens eine temperaturabhängige physikalische oder Verhaltenscharakteristik aufweist;
b) einem Formgedächtnispolymer, das mindestens eine temperaturabhängige physikalische oder Verhaltenscharakteristik aufweist;
c) einem superelastischen Material;
d) einem Mehrstrangseil; und
e) einem hochfesten Polymer.

4. Implantatvorrichtung gemäß einem der vorhergehenden Ansprüche, wobei das Implantat in eine längliche Form, die eine Breite von ungefähr 75% oder weniger seiner entspannten Breite hat, gebogen und verformt werden kann, ohne dass dazu Werkzeuge benötigt werden und ohne dass es dabei beschädigt wird.

5. Implantatvorrichtung gemäß einem der vorhergehenden Ansprüche, wobei das obere längliche Verstärkungselement (932), das mit dem bogenförmigen oberen Rand (930) gekoppelt oder in diesen eingebettet ist, mindestens ein Ende hat, das direkt an einem Knochen oder weichem Gewebe verankert werden kann.

6. Implantatvorrichtung gemäß einem der vorhergehenden Ansprüche, wobei das obere längliche Verstärkungselement (932), das mit dem bogenförmigen oberen Rand (930) gekoppelt oder in diesen eingebettet ist, mindestens ein Ende hat, das mit dem länglichen unteren Verstärkungselement (922) gekoppelt ist.

## Revendications

1. Dispositif d'implant pour la réparation de cartilage endommagé dans une articulation d'un mammifère, ledit dispositif d'implant étant un implant de ménisque (900) dimensionné et façonné pour remplacer le cartilage du ménisque, comprenant:
une surface inférieure lisse et lubrifiée (906) dimensionnée et formée pour s'articuler en contact avec un plateau tibial, ladite surface inférieure (906) étant limitée et définie par un bord intérieur arqué, un bord périphérique arqué (920) et des pointes opposées (908, 909) où ledit bord intérieur arqué et ledit bord périphérique arqué se rencontrent,
une surface d'articulation supérieure lisse et lubrifiée (902), dimensionnée et formée pour s'articuler en contact avec un patin fémoral, après l'implantation ;
une surface périphérique arquée (904) dimensionnée et formée pour contacter le tissu mou dans une capsule du genou, après l'implantation ; et
un bord supérieur arqué (930) où ladite surface d'articulation supérieure (902) et ladite surface périphérique arquée (904) se rencontrent ;
au moins un élément de renforcement allongé inférieur (922) intégré dans ledit bord périphérique arqué (920) ; et,
au moins un composant d'ancrage tissulaire couplé au dit élément de renforcement allongé (922),
**caractérisé en ce que**
un élément de renforcement allongé supérieur (922), auquel sont couplés une pluralité de composants d'ancrage tissulaire, est couplé au dit bord supérieur arqué (930) ou intégré dans celui-ci.

2. Dispositif d'implant selon la revendication 1, dans lequel ledit ou lesdits composants d'ancrage tissulaire sont sélectionnés dans un groupe constitué de :
a. une saillie qui s'étendra dans un trou préparé dans une surface osseuse ;
b. un composant qui peut être fixé à une tête d'une vis à os ;
c. un ou plusieurs brins de fil ;
d. ou un plusieurs brins d'un matériau de suture ;
e. un segment de treillis de renforcement qui est partiellement intégré à l'intérieur du composant polymère moulé et qui s'étend hors dudit composant polymère moulé à au moins un emplacement ; et,
f. au moins un dispositif d'oeillet, chacun doté d'une ouverture.

3. Dispositif d'implant selon l'une quelconque des revendications précédentes, dans lequel au moins un desdits éléments de renforcement (922, 932) est fabriqué en un matériau sélectionné dans le groupe constitué de :
a. un alliage à mémoire de forme ayant au moins une caractéristique physique ou comportementale dépendant de la température ;
b. un polymère d'alliage à mémoire de forme ayant au moins une caractéristique physique ou comportementale dépendant de la température ;
c. un matériau super-élastique ;
d. un câble à brins multiples ; et
e. un polymère haute résistance.

4. Dispositif d'implant selon l'une quelconque des revendications précédentes, dans lequel l'implant peut être fléchi et déformé, sans nécessiter d'outil et sans être endommagé, en une forme allongée ayant une largeur d'environ 75 % ou moins de sa largeur relâchée.

5. Dispositif d'implant selon l'une quelconque des revendications précédentes, dans lequel ledit élément de renforcement allongé (932) qui est couplé au dit bord supérieur arqué (930) ou intégré dans celui-ci a au moins une extrémité qui peut être ancrée directement dans un os ou un tissu mou.

6. Dispositif d'implant selon l'une quelconque des revendications précédentes, dans lequel ledit élément de renforcement allongé supérieur (932) qui est couplé au dit bord supérieur arqué (930) ou intégré dans celui-ci a au moins une extrémité qui est couplée au dit élément de renforcement inférieur allongé (922).
